# EUROPEAN PATENT APPLICATION

(11) **EP 1 695 700 A1**
(43) Date of publication of application: **30.08.2006**
(21) Application number: 05004377.7
(22) Date of filing: 28.02.2005
(51) Int. Cl.: A61K 9/28, A61K 31/485, A61P 25/04

(54) **Dosage form containing oxycodone and naloxone**

(71) Applicant: Euro-Celtique S.A., 2330 Luxembourg (LU)
(72) Inventor: Leyendecker, Petra, 35578 Wetzlar (DE); Hopp, Michael, 65428 Rüsselsheim (DE); Smith, Kevin, Cambs CB4 9LN (GB)
(74) Representative: Maiwald, Walter

(57) **Abstract**

The present invention concerns a dosage form comprising oxycodone and naloxone which characterized by specific *in vivo* parameters such as tₘₐₓ, Cₘₐₓ, AUCt value, mean bowel function score and/or duration of analgesic efficacy.

## Description

### FIELD OF THE INVENTION

The invention concerns a dosage form comprising oxycodone and naloxone which is characterized by specific *in vivo* parameters such as tₘₐₓ, Cₘₐₓ, AUCt value, mean bowel function score and/or duration of analgesic efficacy.

### BACKGROUND OF THE INVENTION

The treatment of severe pain resulting from diseases such as cancer, rheumatism and arthritis is central to the treatment of these diseases. The range of pain felt by tumor patients comprises pain of the periosteum and of the bone itself, as well as visceral pain and pain in soft tissues. All such pain forms render the daily life of patients intolerable and often lead to depressive states. Successful pain therapy resulting in a lasting improvement of quality of life for the patients is therefore equally important for the success of a comprehensive therapy, as is the treatment of the actual causes of the disease.

Having regard to the importance of a successful pain therapy, the World Health Organization (WHO) has developed a 4-step model for the treatment of patients with tumor pain. This model has proven to be effective in daily routine practice and can be extended to patients suffering from chronic pain or pain forms resulting from diseases other than cancer. Depending on the intensity, kind and localization of pain, four steps are distinguished during this therapy, with each next step being indicated if the effect of the pain relief agent used until then is no longer sufficient (Ebell, H. J.; Bayer A. (Ed.): Die Schmerzbehandlung von Tumorpatienten, Thieme 1994 (Supportive Maßnahmen in der Onkologie, Band 3) and Zech, D.; Grond, S.; Lynch, J.; Hertel, D.; Lehmann, K.: Validation of World Health Organisation Guidelines for Cancer Pain Relief: a 10-year prospective study, Pain (1995), 63, 65-76).

According to this 4-step model of the WHO, opioid analgesics take a central role in treating pain. The group of opioid analgesics comprises, besides morphine (which represents the prototype of these pharmaceutically active agents), also oxycodone, hydromorphone, nicomorphine, dihydrocodeine, diamorphine, papaveretum, codeine, ethylmorphine, phenylpiperidine and derivatives thereof; methadone, dextropropoxyphene, buprenorphine, pentazocine, tilidine, tramadol and hydrocodone. The ATCC-Classification (Anatomical Therapeutic Chemical Classification) of the WHO indicates whether the pharmaceutically active agent is an opiod analgesic or not. The pronounced pain-relieving effect of opioid analgesics is due to the imitation of the effect of endogenous, morphine-like acting substances ("endogenous opioids"), whose physiological function is to control the reception and processing of pain stimuli.

Opioids repress the propagation of pain stimuli. Besides the immediate inhibition of neuronal excitatory signal transduction in the spinal cord caused by opioids, an activation of those nerve tracts projecting from the brainstem into the spinal cord also plays a role. This activation results in an inhibition of pain propagation in the spinal cord. Moreover, opioids limit the pain reception of the thalamus and, by affecting the limbic system, they influence the affective pain evaluation.

Opioid receptors are found at different sites in the body. Receptors of the intestine and brain are of particular importance for pain therapy by opioids, especially as their occupation results in different side effects.

Opioid analgesics are considered to be strong agonists if they bind with high affinity to opioid receptors and induce a strong inhibition of pain reception. Substances that also bind with high affinity to opioid receptors, but that do not cause a reduction of pain reception and which thereby counteract the opioid agonists, are designated as antagonists. Depending on the binding behaviour and the induced activity, opioids can be classified as pure agonists, mixed agonists/antagonists and pure antagonists. Pure antagonists comprise, for example, naltrexone, naloxone, nalmefene, nalorphine, nalbuphine, naloxoneazinen, methylnaltrexone, ketylcyclazocine, norbinaltorphimine, naltrindol, 6-β-naloxol und 6-β-naltrexol (Forth W.; Henschler, D.; Rummel W.; Starke, K.: Allgemeine und Spezielle Pharmakologie und Toxikologie, 7. Auflage, 1996, Spektrum Akademischer Verlag, Heidelberg Berlin Oxford).

Due to their good analgesic efficiency, compounds such as oxycodone, tilidine, buprenorphine und pentazocine, have been used in the form of medicaments for pain therapy. It has been proven that medicaments such as Oxigesic® having oxycodone as the analgesic active compound und Valoron® having tilidine as the analgesic active compound are valuable for pain therapy.

However, use of opioid analgesics for pain therapy might be accompanied by undesirable side effects. For instance, long-term use of opioid analgesics can lead to psychological and physical dependence.

Especially the physical dependence of patients suffering from pain on opioid analgesics may lead to the development of tolerance, meaning that upon extended intake, increasingly higher doses of the pain-relieving agent have to be taken by the patient, in order to experience pain relief. The euphoregenic effect of opioid analgesics may lead to the abuse of pain-relievers. Drug abuse and psychological dependence are known, especially among teenagers. However, opioid analgesics are legitimately used for medical purposes and medicine cannot do without them.

Besides the mentioned disadvantages, the use of potent opioid analgesics for pain therapy often also lead to undesirable side effects, such as constipation, breath depression, sickness and sedation. Less frequently, urge or inability to pass water are observed.

Different attempts have been made to counteract the habituation processes and the other side effects occurring during pain therapy. This can be done, e.g. by traditional treatment methods. In the case of drug addiction this might be a drug withdrawal treatment, and in the case of constipation, this might be done by administration of laxatives.

Other attempts aim at minimizing the addictive and habituation forming potential of opioid analgesics, as well as their other side effects by the administration of antagonists which counteract the opioid analgesic. Such antagonists might be naltrexone or naloxone.

There have been numerous proposals and suggestions as to how the application of the aforementioned active compounds could be used to avoid undesired habituation and dependence, or even addiction.

US 3,773,955 and US 3,966,940 suggested formulating analgesics in combination with naloxone, purportedly to prevent dependence-promoting effects such as euphoria and the like upon parenteral application. The avoidance of side effects such as constipation was not addressed.

To limit the parenteral abuse of oral application forms, US 4,457,933 suggested using a combination of morphine with naloxone in defined ranges. The avoidance of side effects such as constipation was not mentioned in this patent either.

US Patent No. 4,582,835 describes, again in order to avoid abuse, a preparation comprising a combination of buprenorphine and naloxone to be administered either parenterally or sublingually.

EP 0 352 361 A1 concerns the treatment of constipation during pain therapy by the oral application of an opioid analgesic and one antagonist. Avoidance of abuse of the opioid analgesic is not an issue in this application.

DE 43 25 465 A1 also concerns the treatment of constipation during pain therapy using a preparation comprising an opioid analgesic and an antagonist. According to this disclosure, the antagonist, which can be naloxone, may be present in higher amounts than the opioid analgesic, which is preferably morphine. The avoidance of abuse of the opioid analgesic is not an issue in DE 43 25 465 A1.

In order to avoid side effects such as constipation and breath depression during pain therapy, preparations have been introduced on the market which can be taken orally and comprise an opioid analgesic and the opioid antagonist, naloxone. The medicament Talwin® of Windrop/Sterling comprises pentazocine and naloxone. The medicament Valoron® of Gödeke comprises a tilidine-naloxone combination.

Besides potent analgesic effect, the reduction of addictive potential and the avoidance of side effects, medicaments suitable for a successful pain therapy should possess additional characteristics.

Generally, medicaments have to be formulated in such a way that the active compounds are stable as long as possible under standard storage conditions. Medicaments have also to be formulated in such a way that the intended release profiles of the active compounds do not change upon long-term storage.

Medicaments suitable for pain therapy should either contain the active compounds in such amounts, or be formulated in such a way, that they have to be taken by the patients only at long intervals. The easier the application scheme for a pain-reliever is, and the clearer it is for the patient why and how often he should take which tablet, the more exactly will he adhere to the physician's orders. The necessity to take the pain-reliever only infrequently will result in increased willingness of the patient to take the pain-reliever (compliance).

The medicament Oxygesic® is a preparation from which the opioid analgesic oxycodone is released in a sustained manner. Oxygesic® does not contain opioid antagonists.

According to EP 0 352 361 A1, neither the opioid analgesic nor the antagonist are formulated to be released in a sustained manner. Accordingly, the time period during which such preparations are effective is limited and preparations have to be taken a number of times a day. The desired compliance of the patient is not achieved. EP 0 352 361 A1 also does not disclose the advantages of formulations of preparations that are characterized by a time-stable and independent release of the active compounds. The storage stability of such preparations is also not addressed by this disclosure.

DE 43 25 465 A1 discloses formulations according to which constipation occurring during pain therapy is prevented by the sustained release of the opioid agonist, while the antagonist, which is present in excess, is not released in a sustained manner. Due to the high first-pass-effect of naloxone, relatively large amounts of this compound have therefore to be used. However, DE 43 25 465 A1 does not disclose preparations, which are characterized by time-stable and independent release of the active compounds. The storage stability of such preparations is also not described therein.

Under the trademark Valoron®, a pain-reliever is marketed which comprises a tilidine naloxone combination. According to the product literature, a formulation is used from which both active compounds are released in a sustained manner. The matrix used comprises a significant amount of water-swellable material, that is HPMC. However, this formulation, given identical mass ratio but different absolute amounts of tilidine and naloxone, shows different release profiles. The release rates of the agonist and the antagonist are not independent from each other. Accordingly, it is necessary for the physician to carry out extensive titration experiments for each individual patient if an increase of the dosage is desired, even though the mass ratio of tilidine:naloxone is not altered, since it cannot be assumed that the release profiles of both components will remain constant. The range of therapeutically suitable amounts of the analgesic is therefore limited.

WO 03/084520 describes a storage-stable pharmaceutical preparation comprising oxycodone and naloxone for use in pain therapy, with the active compounds being released from the preparation in a sustained, invariant and independent manner.

There is a need for oxycodone naloxone dosage forms characterized *by in vivo* parameters which provide for a fast and long-lasting analgesic effect while preventing and/or treating side effects during pain therapy and also preventing or reducing drug abuse.

### SUMMARY OF THE INVENTION

It is therefore an object of the present invention to provide an oxycodone naloxone dosage form which provides a fast analgesic effect and, at the same time, is suitable in chronic maintenance therapy.

It is a further object of the present invention to provide an oxycodone naloxone dosage form which is suitable for the prevention and/or treatment of side effects during pain therapy such as constipation without substantially reducing the analgesic effect of oxycodone.

Further, it is an object of the present invention to provide an oxycodone naloxone dosage form which is suitable to prevent habituation and/or addiction-promoting effects during pain therapy without substantially reducing the analgesic effect of oxycodone.

In particular, it is an object of the present invention to provide a dosage form for pain therapy that, besides high analgesic activity, is characterized by reduced abuse potential and reduced side effects, said dosage form also being characterized by reduced administration frequency thus ensuring increased patient compliance, as well as facilitating individual adaptation of the dosage for each patient.

It is another object of the present invention to provide a sustained release oxycodone naloxone formulation which may also be used to titrate a patient receiving oxycodone therapy and, at the same time, is suitable in chronic maintenance therapy after titration of the patient.

The feature combination of the independent claims serves to attain these and further objects which can be gathered from the following description of the invention. Preferred embodiments of the invention are defined in the dependent claims.

In one aspect of the present invention, a dosage form is provided which comprises oxycodone and naloxone and provides a mean tₘₐₓ for oxycodone at about 1 to about 17 hours, at about 2 to about 15 hours, at about 3 to about 8 hours or at about 4 to about 5 hours after administration at steady state or of a single dose to human patients.

In a further aspect of the present invention, a dosage form is provided which comprises oxycodone and naloxone and provides an improvement of bowel function during pain therapy, in particular compared to administering oxycodone alone.

In a further aspect of the present invention, a dosage form is provided which comprises oxycodone and naloxone and provides an analgesic effect for at least about 12 hours or at least about 24 hours after administration at steady state or of a single dose to human patients.

In a further aspect of the present invention, a dosage form is provided which comprises oxycodone and naloxone and provides an mean AUCt value for oxycodone of about 100 ng·h/mL to about 600 ng·h/mL, or of about 300 ng·h/mL to about 580 ng·h/mL or of about 400 ng·h/mL to about 550 ng·h/mL, or of about 450 ng·h/mL to about 510 ng·h/mL after administration at steady state or of a single dose to human patients.

In a further aspect of the present invention, a dosage form is provided which comprises oxycodone and naloxone and which provides a mean Cₘₐₓ for oxycodone of about 5 ng/mL to about 50 ng/mL, or of about 20 ng/mL to about 40 ng/mL or of about 30 ng/mL or of about 35 ng/mL after administration at steady state or of a single dose to human patients.

According to a further aspect of the present invention, a method of treating moderate to severe pain in a patient by administering a dosage form according to the present invention is provided.

According to another aspect of the invention, a method of treating moderate to severe pain and/or preventing and/or treating side effects during pain therapy, such as constipation, administering a dosage form according to the present invention is provided.

According to further aspect of the present invention, a method of treating moderate to severe pain in a patient while preventing or reducing abuse by administering a dosage form according to the present invention is provided.

### BRIEF DESCRIPTION OF THE DRAWINGS

Figure 1 shows a paper form for assessing the bowel function index (BFI3) which is suitable for use in a method for assessing bowel function.
Figure 2 shows a circular bowel function index (BFI3) meter which is suitable for use in a method for assessing bowel function.
Figure 3 shows the schematic study design for the clinical study of example 1.
Figures 4 to 6 are tables summarizing the values for mean bowel function at each study visit by dose ratio, by absolute dose of naloxone and by absolute dose of naloxone given the same oxycodone/naloxone dose ratio in the ITT population according to example 1.
Figure 7 is a table summarizing the test for difference for each dose of naloxone versus placebo according to example 1.
Figure 8 shows a surface plot of the whole dose range investigated based on the RSREG estimations of the model parameters according to example 1.
Figure 9 shows a contour plot of the bowel function with a granulation of 10 according to example 1.
Figures 10 to 16 show mean observed plasma concentration - time curves for oxycodone, naloxone-3-glucuronide, naloxone, noroxycodone, oxymorphone, noroxymorphone and 6-β-naloxol according to example 2.

### DETAILED DESCRIPTION OF THE INVENTION

Oxycodone is an opioid analgesic that was introduced into the German market as a controlled-release formulation (Oxygesic®) in 1998. Its indication is severe to most severe pain of malignant and non-malignant origin. However, like all opioids, oxycodone has a potential for abuse. The restriction on narcotic drugs worldwide limits the use of opioids in the medical field and impedes the pain therapy of chronic pain patients with strong opioids. According to the present invention, development of habituation and addiction as well as obstipation and breath depression are to be considered as side effects of analgesically effective opioid agonists such as oxycodone.

Naloxone is a commercially available intravenous narcotic antagonist, which is indicated for the blockade of exogenously administered opioids. It acts at all opioid receptor sites (µ, κ, and δ). Following oral administration, naloxone is rapidly absorbed (within 5-30 minutes) but has a very low oral bioavailability of <3% due to an extensive first-pass-metabolism. In low oral doses, naloxone does not become systemically available but acts mainly on local opioid receptors in the gastrointestinal tract.

According to the present invention, severe to moderate pain can be treated by administering an oxycodone/naloxone dosage form according to the present invention while preventing and/or treating side effects during pain therapy, such as constipation and/or while preventing or reducing the abuse of the medicament. In particular embodiments, the dosage forms according to the present invention eliminate the need to first titrate a patient on an immediate release oxycodone dosage form before switching the patient to a sustained release dosage form for chronic therapy.

Co-administration of oxycodone with naloxone by administering dosage forms according to the present invention confers advantages with regard to some of the side effects of the drug. An oxycodone/naloxone dosage form according to the present invention reduces the frequency and intensity of constipation as compared to oxycodone alone. Moreover, an oxycodone/naloxone dosage form according to the present invention reduces oral, intranasal, and i.v. abuse of oxycodone. Since naloxone is not expected to enter the brain, the dosage forms according to the present invention do not inhibit the pain relieving action of the oxycodone. The amount of naloxone in the combination product is preferably high enough to precipitate withdrawal effects or at least strong dislike feelings.

The concentration gradients or blood plasma curves can be described by the parameters such as Cₘₐₓ, tₘₐₓ and AUC. These parameters are important in describing the pharmacokinetic properties of a specific drug formulation.

The Cₘₐₓ value indicates the maximum blood plasma concentration of the active agents, i.e. oxycodone and/or naloxone.

The tₘₐₓ value indicates the time point at which the Cₘₐₓ value is reached. In other words, tₘₐₓ is the time point of the maximum observed plasma concentration. Usually, the blood concentration gradients with a late tₘₐₓ were aimed at for sustained release formulations, because it was assumed that only in that way a prolonged effect could be guaranteed. However, a disadvantage of a late tₘₐₓ value may be the long time period needed in order to achieve an analgesic effect.

The AUC (Area Under the Curve) value corresponds to the area of the concentration curve. The AUC value is proportional to the amount of active agents, i.e. oxycodone and naloxone absorbed into the blood circulation in total and is hence a measure for the bioavailability.

The AUCt value is the value for the area under the plasma concentration-time curve from the time of administration to the last measurable concentration. AUCt are usually calculated using the linear trapezoidal method. Where possible, LambdaZ, which is the terminal phase rate constant, is estimated using those points determined to be in the terminal lock-linear phase. t1/2Z, which is the apparent terminal phase half-life, is commonly determined from the ratio of ln2 to LambdaZ. The areas under the plasma concentration-time curve between the last measured point and infinity may be calculated from the ratio of the final observed plasma concentration (Cₗₐₛₜ) to LambdaZ. This is then added to the AUCt to yield AUCinf, which is the area under the plasma concentration-time curve from the time of administration to infinity.

Parameters describing the blood plasma curve can be obtained in clinical trials, first by once-off administration of the active agent such as oxycodone and naloxone to a number of test persons. The blood plasma values of the individual test persons are then averaged, e.g. a mean AUC, Cₘₐₓ and tₘₐₓ value is obtained. In the context of the present invention, pharmacokinetic parameters such as AUC, Cₘₐₓ and tₘₐₓ refer to mean values. Further, in the context of the present invention, *in vivo* parameters such as values for AUC, Cₘₐₓ, tₘₐₓ, bowel function or analgesic efficacy refer to parameters or values obtained after administration at steady state or of a single dose to human patients.

Pharmacokinetic parameter calculations may be performed with WinNonlin Enterprise Edition, Version 4.1.

The term "bioavailability" is defined for purposes of the present invention as the extent to which active agents such as oxycodone and naloxone are absorbed from the unit dosage forms.

The term "sustained release" is defined for purposes of the present invention as the release of oxycodone and/or naloxone at such a rate that blood levels are maintained within the therapeutic range but below toxic levels over a period of time of about 8 hours or about 12 hours or about 24 hours or even longer.

The phrase "(initial) rapid rate of rise" with regard to oxycodone blood plasma concentration is defined for purposes of the present invention as signifying that the minimum effective analgesic concentration is quickly approached in patients who have measurable if not significant pain at the time of dosing. In particular, this might be achieved by administering a dosage form according the present invention which provides a tₘₐₓ of up to 17 hours, preferably of up to 10 hours, more preferably of up 6 hours or even less, e.g. up to 5 hours or up to 4 hours or up to 3 hours.

The term T_{1/2} is defined for purposes of the present invention as the amount of time necessary for one half of the absorbable dose of oxycodone and/or naloxone to be transferred to plasma. This value may be calculated as a "true" value (which would take into account the effect of elimination processes), rather than an "apparent" absorption half-life.

The term "steady state" means that a plasma level for a given drug has been achieved and which is maintained with subsequent doses of the drug at a level which is at or above the minimum effective therapeutic level and is below the minimum toxic plasma level for oxycodone. For opioid analgesics such as oxycodone, the minimum effective therapeutic level will be partially determined by the amount of pain relief achieved in a given patient. It will be well understood by those skilled in the medical art that pain measurement is highly subjective and great individual variations may occur among patients. It is clear that after the administration of each dose the concentration passes through a maximum and then again drops to a minimum.

The steady state may be described as follows: At the time t = 0, the time the first dose is administered, the concentration C is also 0. The concentration then passes through a first maximum and then drops to a first minimum. Before the concentration drops to 0, another dose is administered, so that the second increase in concentration doesn't start at 0. Building on this first concentration minimum, the curve passes through a second maximum after the second dose has been administered, which is above the first maximum, and drops to a second minimum, which is above the first minimum. Thus, the blood plasma curve escalates due to the repeated doses and the associated step-by-step accumulation of active agent, until it levels off to a point where absorption and elimination are in balance. This state, at which absorption and elimination are in equilibrium and the concentration oscillates constantly between a defined minimum and a defined maximum, is called steady state.

The terms "maintenance therapy" and "chronic therapy" are defined for purposes of the present invention as the drug therapy administered to a patient after a patient is titrated with an opioid analgesic to a steady state as define above.

In the context of the present invention, "agonist" or "analgesic" always refers to oxycodone and "antagonist" always refers to naloxone. Active compounds according to the present invention are oxycodone and/or naloxone and/or pharmaceutically acceptable salts thereof. Unless expressly indicated otherwise, amounts and ratios of the active compounds as described herein refer to the form actually used, i.e. the free base or a pharmaceutically acceptable salt thereof. Further, unless expressly indicated otherwise, amounts and ratios of the active compounds as described herein refer to the anhydrous form of the compound.

In one aspect, the present invention provides a dosage form comprising oxycodone and naloxone which provides a mean tₘₐₓ for oxycodone at about 1 to about 17 hours, at about 2 to about 2 to about 15 hours, at about 3 to about 8 hours or at about 4 to about 5 hours after administration at steady state to human patients. Mean tₘₐₓ values of oxycodone of about 6, about 7, about 9, about 10, about 11, about 12, about 13, about 15, about 16 hours or more are also preferred.

Preferably, or alternatively, the oxycodone naloxone dosage forms according to the present invention provide an improvement of the bowel function during pain therapy. In the context of the present invention, an improvement of bowel function during pain therapy usually means that bowel function is improved compared to the administration of oxycodone alone, e.g. in combination with naloxone placebo.

Bowel function is usually assessed by observing parameters which are associated with bowel function. In particular, bowel function may be determined based on parameters selected from ease or difficulty of defecation, feeling of incomplete bowel evacuation, and/or personal judgment of patient regarding constipation. Other parameters which may be observed alternatively or in addition in order to assess the bowel function of a patient include among other things stool frequency, stool consistency, cramping, and painful laxation.

It is preferred to determine bowel function by measuring parameters which are associated with bowel function using numerical analog scales (NAS) for these parameters since this may provide more accurate results. This is particularly advantageous when assessing the bowel function in patients receiving treatment with analgesics, since analgesic efficacy of drugs is usually assessed using a numeric analog scale. Hence, patients receiving treatment with analgesics are used to handle numerical analog scales which provides for obtaining meaningful results.

In a preferred embodiment, the oxycodone/naloxone dosage forms according to the present invention provide an improvement of the bowel function characterized by an improvement of the mean bowel function score of at least 5, at least about 8, at least about 10 or at least about 15 after administration at steady state to human patients, wherein the mean bowel function score is measured with a numerical analog scale ranging from 0 to 100.

The mean bowel function score is in particular determined by a method for assessing bowel function in a patient which comprises the following steps:
- providing the patient with a numeric analog scale for at least one parameter, which parameter is associated with bowel function;
- causing the patient to indicate on the numeric analog scale the amount and/or intensity of the parameter being experienced; and
- observing the amount and/or intensity of the at least one parameter indicated on the numeric analog scale in order to assess bowel function.

The patient usually indicates the amount and/or intensity of parameter being experienced during the last days or weeks, e.g. during the last 1, 2, 3, 4, 5, 6, 7, 10 or 14 days.

The numerical analog scale on which the patient indicates his/her subjective experience of the observed parameter may have any size or form and may range from 0 or any other number to any number, such as from 0 to 10 or from 0 to 50 or from 0 to 300 or from 1 to 10.

If more than one parameter is observed, a mean bowel function may be obtained in form of a numerical value which is the mean of the parameters observed, e.g. the three numeric analog scale values for ease or difficulty of defecation, feeling of incomplete bowel evacuation and judgment of constipation. The mean bowel function is also designated as mean bowel function score, bowel function index or BFI3 (if three parameters are observed).

In particular, bowel function may be determined based on the following three parameters:
- ease or difficulty of defecation, for example during the last 7 days according to the patient assessment, wherein 0 corresponds to no difficulties and 100 corresponds to severe difficulties;
- feeling of incomplete bowel evacuation, for example during the last 7 days according to the patient assessment, wherein 0 corresponds to no feeling of incomplete bowel evacuation and 100 corresponds to very strong feeling of incomplete bowel evacuation;
- personal judgment of patient regarding constipation, for example during the last 7 days, wherein 0 corresponds to no constipation at all and 100 corresponds to very heavy constipation.

Mean bowel function may be obtained in form of a numerical value which is the mean of the parameters observed, e.g. the three numeric analog scale values for ease or difficulty of defecation, feeling of incomplete bowel evacuation and judgment of constipation.

In particular, the method for assessing bowel function is performed by using devices or analog scales as described in the following.

In one embodiment, the parameter scale or numeric analog scale presented to the patient may be an uninterrupted line that bears no indicators or markings other than at the ends indicating no experience or very strong experience of the parameter to be observed. The patient is then caused to indicate the amount and/or intensity of the parameter experienced by making a dash on the uninterrupted line. Then, the health care provider or medical practitioner may measure the distance from the dash to the end indicating no experience or to the end indicating very strong experience, and divide this measure by the distance between both ends. The result is a numerical value which is a score for the bowel function. If more than one parameter is observed a mean bowel function score is usually determined by averaging the numeric analog scale values for each parameter. If three parameters are observed this mean bowel function score is also designated as Bowel Function Index or BFI3. Rome 11-criteria can be detected by this scale.

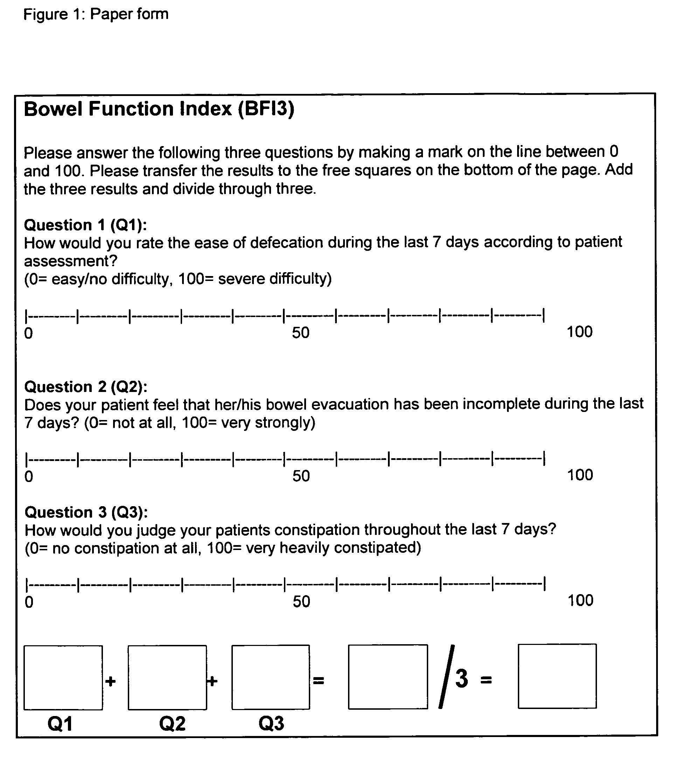

In a further embodiment, Fig. 1 illustrates an example for a paper form which can be used for assessing the bowel function index or mean bowel function score. In particular, the patient or the medical practitioner responsible for this patient may be asked to answer questions rendered on the paper form which concern parameters associated with bowel function such as the ease or difficulty of defecation, for example during the last 1, 3, 7 or 14 days; the feeling of incomplete bowel evacuation, for example during the last 1, 3, 7 or 14 days; and a personal judgment of the patient regarding constipation, again for example during the last 1, 3, 7 or 14 days. In this embodiment, the questions are answered by making a mark on a line between 0 and 100, wherein 0 corresponds to no difficulties and 100 corresponds to severe difficulties of defecation and/or wherein 0 corresponds to no feeling of incomplete bowel evacuation at all and 100 corresponds to very strong feeling of incomplete bowel evacuation and/or wherein 0 corresponds to no constipation at all and 100 corresponds to very heavy constipation. Of course, the scale may range from 0 or any other number to any number, such as from 0 to 10 or 0 to 50 or 0 to 300 or 1 to 10. The three numerical values which, for example, may be obtained by measuring the distance from the mark to the end indicating no experience or to the end indicating very strong experience, and dividing this measure by the distance between both ends, are then preferably added and divided by three in order to obtain the mean bowel function score or mean bowel function index (BFI) or BFI3.

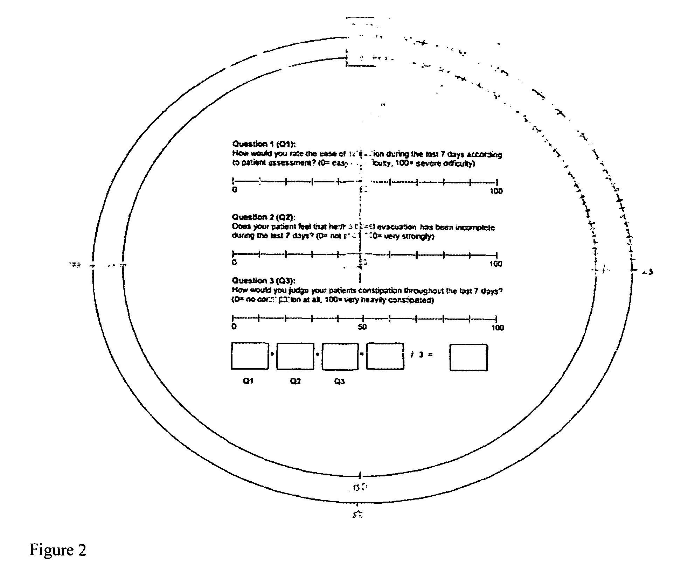

In a further embodiment, Fig. 2 illustrates an example of a circular BFI meter for determining the mean bowel function score. Preferably, a circular BFI meter contains a paper form with questions concerning the patient's assessment on one or more parameters which are associated with bowel function as described above. Further, such a circular BFI meter preferably contains a numerical scale on an inner circle and a numerical scale on an outer scale. The numerical scales are preferably correlated with each other such that a value on one scale is a multiple of the corresponding value on the other scale wherein the factor corresponds to the number of parameters which are observed. For example, if three parameters are observed, a value on one scale shows the corresponding value on the other scale divided or multiplied by three. Moreover, a BFI meter contains a needle or pointer which is attached to the middle of the circle and can be moved around the circle in order to facilitate the correlation of the corresponding values on the numerical scales on the inner and outer circle.

For example, three questions concerning the ease or difficulty of defecation, for example during the last 7 days, wherein 0 corresponds to no difficulties and 100 corresponds to severe difficulties; the feeling of incomplete bowel evacuation, for example during the last 7 days according to the patient assessment, wherein 0 corresponds to not at all and 100 corresponds to very strong; and a personal judgment of the patient regarding constipation, in order to obtain the BFI 3 are given on the inner field of a circle of the BFI meter. On the inner circle (3), a scale going clockwise from 0-300 is arranged. On the outer circle (4), a scale going clockwise from 0-100 is arranged which is in line with the marks of the scale of the inner circle and shows the value of the inner circle divided by 3. To facilitate the calculation, a needle or pointer (1) is attached to the middle of the circle which can be moved around the circle. At the outer end of the needle there is a window (2) which frames the numbers of the inner and outer circle. In order to assess the mean bowel function the needle may be moved to the number in the inner circle which is the result of question 1. Then, the result of question 2 may be added by moving the needle to that point of the inner circle. In a third step, the result of question 3 is added by moving the needle to the resulting point of the inner circle. As a result, the mean bowel function score can be seen on the outer circle.

In other preferred embodiments, the method according to the present invention may be performed with analogs scales as described in US 6,258,042 B1 and WO 03/073937 A1 which have to be adapted to devices or analog scales as described above. The disclosures of these two references are hereby incorporated by reference.

Preferably, or alternatively, the oxycodone naloxone dosage forms according to the present invention provide an analgesic effect for at least 8 hours, more preferably for at least 12 hours, or most preferably for at least about 24 hours after administration at steady state or of a single dose to human patients.

Preferably, or alternatively, the oxycodone naloxone dosage forms according to the present invention provide an mean AUCt value for oxycodone of about 100 ng·h/mL or about 200 ng·h/mL or about 300 ng·h/mL to about 600 ng·h/mL, more preferably about 400 ng·h/mL to about 550 ng·h/mL and most preferably from about 450 ng·h/mL to about 510 ng·h/mL. Preferably, these mean AUCt values for oxycodone refer to an oxycodone naloxone dosage forms according to the present invention which comprise 40 mg oxycodone or a pharmaceutically acceptable salt thereof and, e.g., 20 mg naloxone or a pharmaceutically acceptable salt thereof. For oxycodone naloxone dosage forms according to the present invention comprising less than 40 mg oxycodone or a pharmaceutically acceptable salt thereof, the mean AUCt values for oxycodone may be lower such as 50 ng·h/mL or 75 ng·h/mL.

Preferably, or alternatively, the oxycodone naloxone dosage forms according to the present invention provide a mean Cₘₐₓ value for oxycodone of about 5 ng/mL to about 50 ng/mL, more preferably of about 20 ng/mL to 40 ng/mL or most preferably of about 30 ng/mL of about 35 ng/mL. Preferably, these mean Cₘₐₓ values for oxycodone refer to an oxycodone naloxone dosage forms according to the present invention which comprise 40 mg oxycodone or a pharmaceutically acceptable salt thereof and, e.g., 20 mg naloxone or a pharmaceutically acceptable salt thereof. For oxycodone naloxone dosage forms according to the present invention comprising less than 40 mg oxycodone or a pharmaceutically acceptable salt thereof, the mean Cₘₐₓ values for oxycodone may be lower such as 1 ng/mL or 3 ng/mL.

The oxycodone naloxone formulations according to the present invention, which provide an initial rapid rate of rise in the plasma concentration and/or have a tₘₐₓ value e.g. of up to 8 hours, preferably up to 6 hours or up to 5 hours or even up to 4 hours, are advantageous in that a fast and greater analgesic efficacy is achieved. No substantially flat serum concentration curve is exhibited, but instead a more rapid initial opioid release is provided, so that the minimum effective analgesic concentration can be more quickly attained in many patients. This makes the dosage forms according to the present invention also suitable for titrating patients by avoiding the necessity of first titrating on an immediate release oxycodone naloxone dosage form before switching him to a sustained release dosage form for chronic therapy.

Further, there is no significantly greater incidence of side-effects such as constipation which would normally be expected as higher peak plasma concentrations as a result of an initial rapid rate of rise in the plasma concentration occur.

Further, particularly if the dosage form according to the present invention is a matrix formulation, it is ensured that the agonist, i.e. oxycodone, as well as the antagonist, i.e. naloxone, are always released in predetermined percentages and that their release rates do not influence each other. Thereby, abuse of the medicament, which presupposes that oxycodone can selectively be extracted from the formulation, is prevented. The formulation according to the present invention therefore disables selective extraction of oxycodone from the dosage form without the corresponding amount of the antagonist naloxone, regardless of the absolute and relative amounts of agonist and antagonist chosen.

Hence, the dosage forms according to the present invention are also suitable for a method for titrating human patients with a sustained release oxycodone naloxone formulation. The first step of this embodiment comprises administering to a human patient, e.g. on a twice-a-day or once-a-day basis, a unit dose of the sustained release oxycodone/naloxone dosage forms as described above and in the following paragraphs. Thereafter, this embodiment includes the further step of monitoring pharmacokinetic and pharmacodynamic parameters elicited by said formulation in said human patient and determining whether said pharmacokinetic and/or pharmacodynamic parameters are appropriate to treat said patient on a repeated basis. The patient is titrated by adjusting the dose of oxycodone and/or naloxone administered to the patient by administering a unit dose of the dosage forms according to the present invention containing a different amount of oxycodone and/or naloxone if it is determined that said pharmacokinetic and/or said pharmacodynamic parameters are not satisfactory or maintaining the dose of oxycodone and/or naloxone in the unit dose at a previously administered amount if said pharmacokinetic and/or pharmacodynamic parameters are deemed appropriate. The titration is continued by further adjusting the dose of oxycodone and/or naloxone until appropriate steady-state pharmacokinetic/pharmacodynamic parameters are achieved in the patient. Thereafter, the administration of the dose of oxycodone and/or naloxone in the sustained release dosage form according to the present invention is continued, e.g. on a twice-a-day or once-a-day basis, until treatment is terminated.

In a further preferred embodiment, oxycodone and/or naloxone are released from the dosage forms according to the present invention in a sustained, invariant and/or independent manner.

This embodiment ensures that, given identical relative amounts, the active compounds show equal release profiles, independent of the absolute amount present. Such an independent release behaviour provides a wide range of useable absolute amounts of the analgesic active substance to the physician, given that the optimal agonist/antagonist ratio is known. Thus, it is possible to comfortably adjust the dosage for each individual patient, either by a step-wise dosage increase or, if necessary, a step-wise dosage reduction. This ability to adjust the dosage for the individual patient is extremely useful from a medical point of view.

The sustained, invariant and/or independent release of the active compounds, i.e. oxycodone and naloxone or pharmaceutically acceptable salts thereof, ensures additionally that pharmaceutical preparations produced according to the invention are characterized by a low administration frequency, so that high patient compliance is achieved. Furthermore, preparations according to the invention allow the physician to adjust the dosage for individual patients. Preparations according to the invention enable use over a broad range with respect to the useable absolute amounts of the active compounds and ensure that the active compounds, even after long-term storage, become effective with equal release profiles.

According to the present invention, sustained release of oxycodone or a pharmaceutically acceptable salt thereof and/or naloxone or a pharmaceutically acceptable salt thereof means that pharmaceutically active substances are released from the medicament over a longer period of time than they are known from formulations for immediate release.

In a specific embodiment of the present invention, the dosage form release is between 25% to 65%, preferably between 30% to 60%, more preferably between 35% to 55% and even more preferred between 40% to 50% of oxycodone or a pharmaceutically acceptable salt thereof and/or naloxone or a pharmaceutically acceptable salt thereof after 4 hours.

Other specific embodiments of the invention relate to dosage forms that release between 70% to 100%, preferably between 75% to 100%, more preferably between 80% to 95% and even more preferably between 80% to 85%, between 85% to 90% or between 90% to 95% of oxycodone or a pharmaceutically acceptable salt thereof and/or naloxone or a pharmaceutically acceptable salt thereof after 8 hours. Preferred embodiments of the invention also relate to preparations that release approximately 80%, approximately 85%, approximately 90% or approximately 95% of oxycodone or a pharmaceutically acceptable salt thereof and/or naloxone or a pharmaceutically acceptable salt thereof after 8 hours.

According to the invention, dosage forms or formulations of medicaments that ensure such a sustained release of the active compounds from the preparation or dosage form are designated as retard formulations, sustained release formulations or prolonged release formulations. According to the invention, the release of the active compounds preferably occurs in a pH-independent manner. Further, according to the invention, the term "sustained release" refers to the release of active compounds from a medicament over an extended period of time. It does not imply the controlled release at a defined place; therefore, it does not mean that the active compounds are either released only in the stomach, or only in the intestine.

According to the invention, "independent release" means that, given the presence of at least two active compounds, a change of the absolute amount of one compound does not influence the release profiles of the other compounds so that the release profiles of the other compounds are not changed. For dosage forms or formulations according to the invention such an independent release behaviour is independent of the pH value, for which the release is measured, or of the production process. The pH independency particularly applies to the acidic range, i.e. for pH values < 7. The release profile or release behaviour is defined as the change of the release of the active compound from the formulation with time, with the amount of each active compound released provided in percents of the total amount of the active compound.

The release profile may be determined by known tests. Preferably, the release of the active compounds from a sustained release formulation is determined by the Basket Method according to USP at pH 1.2 or pH 6.5 with HPLC.

E.g., this means that the release profile of oxycodone observed for an oxycodone/naloxone-combination with 12 mg oxycodone and 4 mg naloxone does not differ from that of a corresponding preparation with the same formulation containing 12 mg oxycodone and 6 mg naloxone.

In particular, independent release is of interest if the release profile of preparations having substantially equal compositions is compared. Preparations of substantially equal composition have different amounts of the active compounds but are otherwise basically the same with respect the components of the composition which essentially influence the release behaviour.

E.g., if the above-mentioned preparations are compared (with the first preparation comprising 12 mg oxycodone and 4 mg naloxone and the second preparation comprising 12 mg oxycodone and 6 mg naloxone) both preparations, provided that they have the same total weight, will provide for the same release profile for oxycodone and naloxone if the difference in the naloxone amount is replaced by a component in the formulation that typically does not influence the release behaviour.

The person skilled in the art is well aware that if the amount of the active compound in which two dosage forms differ is replaced by a substance that is essential for the release behaviour of the formulation, such as ethylcellulose or a fatty alcohol, differences in the release behaviour may occur. Thus, independent release is preferably provided by dosage forms that have different amounts of the active compounds but are otherwise identical or at least highly similar with respect to the components that essentially influence the release behaviour (given that formulations of the same total weight are compared).

According to the invention, "invariant release behaviour" or "invariant release profile" is defined so that the percentage of the absolute amount of each active compound released per time unit does not significantly change and remains sufficiently constant if the absolute amounts of an active compound is altered. Sufficiently constant percentages mean that the percentage released per time unit deviates from a mean value by not more than 20%, preferably by not more than 15% and especially preferably by not more than 10%. The mean value may be calculated from six measurements of the release profile. Of course, the amount released per time unit has to satisfy the legal and regulatory requirements.

For example, this means that given an oxycodone/naloxone combination of 12 mg oxycodone and 4 mg naloxone, during the first 4 hours 25% oxycodone and 20% naloxone are released. If the oxycodone/naloxone combination contains 24 mg oxycodone and 8 mg naloxone, again 25% oxycodone and 20% naloxone will be released during the first 4 hours. In both cases the deviation will not be more than 20% from the mean value (which in this example is 25% oxycodone and 20% naloxone).

As outlined for the independent release behaviour, invariant release is of particular interest if preparations of substantially equal composition are compared. Such preparation differ with respect to the amount of the active compounds, but are of the same or at least highly similar composition with respect to the release-influencing components of the preparation. Typically, the difference in the amount of an active compound will be replaced by the amount of a pharmaceutical inert excipient which does not substantially influence the release behaviour of the preparation. Such a pharmaceutical excipient may be lactose, which is a typical filler in pharmaceutical preparations. The person skilled in the art is well aware that invariant release may not be provided by preparations in which the difference in the amount of an active compound is replaced by substances that are known to essentially influence the release behaviour of the preparation, such as ethylcellulose or fatty alcohols.

According to the invention "storage stable" or "storage stability" means that upon storage under standard conditions (at least two years at room temperature and usual humidity) the amounts of the active compounds of a medicament formulation do not deviate from the initial amounts by more than the values given in the specification or the guidelines of the common Pharmacopoeias. According to the invention, storage stability also means that a preparation produced according to the invention can be stored under standard conditions (60% relative humidity, 25°C) as it is required for admission to the market.

According to the invention, "storage stable" or "time stable" also means that after storage under standard conditions the active compounds show release profiles as they would upon immediate use without storage. According to the invention, the admissible fluctuations with respect to the release profile are characterized in that the amount released per time unit fluctuates by no more than 20%, preferably no more than 15% and especially preferably no more than 10 %, with respect to a mean value. The mean value is calculated from six measurements of the release profile.

Storage stability is preferably determined by the Paddle Method according to USP at pH 1.2 with HPLC.

According to the invention, a "non-swellable" or "substantially non-swellable" diffusion matrix is a matrix formulation for which the release of the active compounds is not influenced (or at least not to a relevant degree) by swelling of the matrix (particularly in the physiological fluids of the relevant target sites in the patient's body).

According to the invention, the term "substantially non-swellable" diffusion matrix also refers to a matrix whose volume will increase by approximately 300%, preferably by approximately 200%, more preferably by approximately 100%, by approximately 75% or by approximately 50%, even more preferably by approximately 30% or by approximately 20% and most preferably by approximately 15%, by approximately 10%, by approximately 5% or by approximately 1% in aqueous solutions (and particularly in the physiological fluids of the relevant target sites in the patient's body).

Preparations produced according to the invention can be applied orally, nasally, rectally and/or by inhalation for use in pain therapy. According to the invention, parenteral application is not envisaged. Especially preferred is a formulation for oral application.

In one embodiment, oxycodone and/or naloxone are present in the dosage form in form the free base.

In an alternative preferred embodiment, oxycodone and/or naloxone are present in the dosage form in form a pharmaceutically acceptable salt, derivative, and the like. Preferred salts comprise, inter alia, hydrochloride, sulfate, bisulfate, tartrate, nitrate, citrate, bitratrate, phosphate, malate, maleate, hydrobromide, hydroiodide, fumarate, succinate and the like.

Further, it is preferred that the agonist is present in excess of the antagonist. The excess of the agonist is defined based on the amount of the unit dosage of the antagonist present in the combination preparation. The extent of the excess of the opioid agonist is usually given in terms of the weight ratio of agonist to antagonist. Preferred weight ratios of oxycodone or a pharmaceutically active salt thereof and naloxone or a pharmaceutically active salt thereof are 25:1, 15:1, 10:1, 5:1, 4:1, 3:1, 2:1 and 1.5:1.

Further, it is preferred that the dosage forms according to the present invention comprise from 10 mg to 150 mg oxycodone or a pharmaceutically active salt thereof and more preferred from 20 mg to 80 mg oxycodone or a pharmaceutically active salt thereof and/or from 1 mg to 50 mg naloxone or a pharmaceutically active salt thereof and more preferred from 5 mg to 20 mg naloxone or a pharmaceutically active salt thereof per unit dosage. In other preferred embodiments of the invention, the dosage forms or preparations may comprise from 5 to 50 mg oxycodone or a pharmaceutically active salt thereof, from 10 to 40 mg oxycodone or a pharmaceutically active salt thereof, from 15 to 30 mg oxycodone or a pharmaceutically active salt thereof or approximately 20 mg oxycodone or a pharmaceutically active salt thereof. Preferred dosage forms of the invention may also comprise from 1 to 40 mg naloxone or a pharmaceutically active salt thereof, 5 to 30 mg naloxone or a pharmaceutically active salt thereof, or 10 to 20 mg naloxone per unit dosage or a pharmaceutically active salt thereof.

Matrix-based retardation formulations may preferably be used as dosage forms or formulations in accordance with the invention. It is especially preferred that the dosage forms are based on a substantially non-swellable diffusion matrix.

Preferably, matrix materials for dosage forms according to the present invention comprise polymers based on ethylcellulose, with ethylcellulose being an especially preferred polymer. Especially preferred do matrices comprise polymers which are available on the market under the trademark Ethocel Standard 45 Premium® or Surelease®. Particularly preferred is the use of ethylcellulose N45 or of Surelease®E-7-7050.

It is particularly preferred that dosage forms according to the present invention comprise ethylcellulose and at least one fatty alcohol as the matrix components that essentially influence the release characteristics of the matrix. The amounts of ethylcellulose and the at least one fatty alcohol may significantly vary so that preparations with different release profiles may be achieved. Even though the inventive preparations usually will comprise both of the afore-mentioned components, in some cases it may be preferred that the preparations comprise only ethylcellulose or the fatty alcohol(s) as the release determining components.

Dosage forms in accordance with the invention may further comprise fillers and additional substances, such as granulating aids, lubricants, dyes, flowing agents and plasticizers.

Lactose, glucose or saccharose, starches and their hydrolysates, microcrystalline cellulose, cellatose, sugar alcohols such as sorbitol or mannitol, polysoluble calcium salts like calciumhydrogenphosphate, dicalcium- or tricalciumphosphat may be used as fillers.

Povidone may be used as granulating aid.

Highly-disperse silica (Aerosil®), talcum, corn starch, magnesium oxide and magnesium stearate or calcium stearate may preferably be used as flowing agents or lubricants.

Magnesium stearate and/or calcium stearate can preferably be used as lubricants. Fatty acids like stearic acid, or fats like hydrated castor oil can also preferably be used.

Polyethylene glycols and fatty alcohols like cetyl and/or stearyl alcohol and/ or cetostearyl alcohol can also be used as additional substances that influence retardation.

If fillers and additional substances such as dyes and the mentioned lubricants, flowing agents and plasticizers are used, care has to be taken that according to the invention only such combinations together with the matrix forming substance and/or the matrix forming substances are used, which ensure *in vivo* parameters of the active compounds in accordance with the invention.

All these additional components of the formulations will preferably be chosen in such a way that the release matrix receives the character of a substantially non-water- or non-buffer-swellable and non-erosive diffusion matrix.

According to the invention, it is especially preferred that the dosage forms comprise ethylcellulose such as ethylcellulose N45 or Surelease® E-7-7050 as a matrix-building substance, stearyl alcohol as fatty alcohol, magnesium stearate as lubricant, lactose as filler and povidone as a granulating aid.

In one embodiment, the dosage form according to the present invention contains oxycodone in an amount corresponding to 20 mg anhydrous oxycodone hydrochloride, and naloxone in an amount corresponding to 10 mg anhydrous naloxone hydrochloride. For those dosage forms containing 20 mg oxycodone hydrochloride and 10 mg naloxone hydrochloride it is especially preferred that the retardant materials are selected from ethyl cellulose and stearyl alcohol. In some specific embodiments, such dosage forms contain at least 29 mg stearyl alcohol or at least 29.5 mg stearyl alcohol, or even at least 30 mg stearyl alcohol. Preferred amounts for ethylcellulose in dosage forms according to this embodiment are at least 8, or at least 10, or at least 12 mg ethylcellulose

In other embodiments, the dosage form contains oxycodone in an amount corresponding to 10 mg anhydrous oxycodone hydrochloride and naloxone in an amount corresponding to 5 mg naloxone hydrochloride. In this embodiment, it is also preferred that the retardant materials are selected from ethylcellulose and stearyl alcohol. Preferred amounts for ethylcellulose and stearyl alcohol in dosage forms according to this embodiment are at least 8, or at least 10, or at least 12 mg ethylcellulose and/or at least 20, or at least 25, or at least 27 mg stearyl alcohol.

In other preferred embodiments, the dosage forms according to the present invention contain oxycodone in an amount corresponding to 40 mg anhydrous oxycodone hydrochloride and naloxone in an amount corresponding to 20 mg anhydrous naloxone hydrochloride. Again, the retardant materials are preferably selected from ethylcellulose and stearyl alcohol. In this embodiment, the dosage forms preferably contain at least 22 mg, or at least 24 mg, or at least 26 mg ethylcellulose and/or at least 55 mg, or at least 59 mg, or at least 61 mg stearyl alcohol. Preferred amounts for ethylcellulose in dosage forms according to this embodiment are at least 8, or at least 10, or at least 12 mg ethylcellulose

Dosage forms in accordance with the invention can be produced like all common dosage forms which, in principle, are suitable for retardation formulations and which provide for the *in vivo* parameters of the active compounds, i.e. oxycodone and naloxone, in accordance with the invention. Especially suitable are tablets, multi-layer tablets and capsules. Additional application forms like granules or powders can be used, with only those applications forms being admissible that provide a sufficient retardation and a release behaviour in accordance with the invention.

Pharmaceutical preparations may also comprise film coatings. However, it has to be ensured that the film coatings do not negatively influence the release properties of the active compounds from the matrix and the storage stability of the active compounds within the matrix. Such film coatings may be colored or may comprise an initial dosage of active compounds if required. The active compounds of this initial dosage will be immediately released so that the therapeutically effective blood plasma level is reached very quickly.

Pharmaceutical preparations or preliminary stages thereof which are in accordance with the invention can be produced by build-up or break-down granulation. A preferred embodiment is the production by spray granulation with subsequent drying of the granules. Another preferred embodiment is the production of granules by build-up granulation in a drum or on a granulating disk. The granules may then be pressed into e.g. tablets using appropriate additional substances and procedures. The person skilled in the art is familiar with granulating technology as applied to pharmaceutical technology.

Production of pharmaceutical preparations or preliminary stages thereof, which are in accordance with the invention, by extrusion technology is especially advantageous. In one preferred embodiment, pharmaceutical preparations or preliminary stages thereof are produced by melt extrusion with co- or counter-rotating extruders comprising two screws. Another preferred embodiment is the production by means of extrusion, with extruders comprising one or more screws. These extruders may also comprise kneading elements.

Extrusion is also a well-established production process in pharmaceutical technology and is well known to the person skilled in the art. The person skilled in the art is well aware that during the extrusion process, various parameters, such as the feeding rate, the screw speed, the heating temperature of the different extruder zones (if available), the water content, etc. may be varied in order to produce products of the desired characteristics.

The aforementioned parameters will depend on the specific type of extruder used. During extrusion the temperature of the heating zones, in which the components of the inventive formulation melt, may be between 40 to 120 °C, preferably between 50 to 100 °C, more preferably between 50 to 90 °C, even more preferably between 50 to 85 °C and most preferably between 65 to 80° C, particularly if counter-rotating twin screw extruders (such as a Leistritz Micro 18 GGL or Leistritz Micro 27 GGL) are used. The person skilled in the art is well aware that not every heating zone has to be heated. Particularly behind the feeder where the components are mixed, cooling at around 25 °C may be necessary. The screw speed may vary between 100 to 500 revolutions per minute (rpm), preferably between 100 to 250 rpm, more preferably between 100 to 200 rpm and most preferably around 150 rpm, particularly if counter-rotating twin screw extruders (such as a Leistritz Micro 18 GGL) are used. The geometry and the diameter of the nozzle may be selected as required. The diameter of the nozzle of commonly used extruders typically is between 1 to 10 mm, preferably between 2 to 8 mm and most preferably between 3 to 5 mm. The ratio of length versus diameter of the screw of extruders that may be used for production of inventive preparations is typically around 40:1.

Generally, the temperatures of the heating zones have to be selected such that no temperatures develop that may destroy the pharmaceutically active compounds. The feeding rate und screw speed will be selected such that the pharmaceutically active compounds are released from the preparations produced by extrusion in a sustained, independent and invariant manner and are storage stable in the matrix. If e.g. the feeding rate is increased, the screw speed may have to be increased correspondingly to ensure the same retardation.

The person skilled in the art knows that all the aforementioned parameters depend on the specific production conditions (extruder type, screw geometry, number of components etc.) and may have to be adapted such that the preparations produced by extrusion provide for *in vivo* parameters of oxycodone in accordance with the present invention.

Examples that display highly advantageous embodiments of the invention are set out below. The examples are not to be interpreted as limiting the possible embodiments of the invention.

### EMBODIMENT EXAMPLES

### Example 1: Optimization of Naloxone - Oxycodone Ratio in Pain Patients

The method for the assessment of bowel function and analog scales for use in this method were employed in a clinical Phase II study conducted in Europe.

The clinical Phase II trial was designed and carried out to investigate whether an oxycodone/naloxone combination would lead to a comparable analgesia with a decrease in constipation in patients with severe chronic pain of tumor and non-tumor origin, and need for laxatives, when compared with oxycodone alone. In addition, analyses were carried out to determine which dose ratio of oxycodone to naloxone was the most effective and most suitable for further development with respect to bowel function improvement, analgesic efficacy, and safety.

### 1. Test Treatment, Dose, and Mode of Administration

Blinded naloxone CR tablets (5 mg and 10 mg) were supplied in bottles. The dosage regimen was constant for the entire double-blind treatment period and no dose adjustments were allowed. Patients received 5, 10 or 20 mg of oral naloxone each morning and evening.

Open label oxycodone CR tablets (10 mg and 20 mg) were supplied in PP blisters. Dose adjustments could be performed during the titration/run-in period and 10 mg CR oxycodone tablets were available as rescue medication throughout the entire study. The dosage regimen was constant for the entire double-blind treatment period. Patients received 20, 30 or 40 mg of oral oxycodone each morning and evening.

Blinded naloxone placebo tablets were optically identical to naloxone tablets 5 mg and 10 mg. Dose and mode of administration were as for naloxone CR tablets.

### 2. Study Design

The clinical study was conducted in Germany as a multi-center, prospective, controlled, randomized, double-blind (with placebo-dummy), four group parallel study with oral controlled release (CR) oxycodone, oral controlled-release (CR) naloxone and corresponding naloxone placebo.

The study had three core phases: a pre-randomization phase, a 4-week double-blind treatment period (maintenance phase) and a follow-up phase. The pre-randomization phase consisted of screening and titration/run-in. Following screening, patients entered either a titration or run-in period. Patients with insufficient pain pre-treatment entered a minimum 2-week titration period and were individually titrated and stabilized at an oxycodone dose of 40 mg, 60 mg or 80 mg per day. Patients on stable oxycodone pre-treatment at screening (between 40-80 mg/day) and with concomitant constipation, entered a 1 week run-in period and were eligible for the maintenance phase without prior titration. For all patients, the dose of oxycodone could be adjusted during titration or run-in and investigators maintained compulsory telephone contact every 2^{nd} day to assess pain control and make dose changes.

At the end of the titration/run-in period, patients who were receiving a stable maintenance dose of 40 mg, 60 mg or 80 mg oxycodone per day (with no more than 5 rescue medication intakes per week) and had a medical need for the regular intake of laxatives were randomized to one of 3 naloxone treatment groups or a naloxone placebo treatment group. Each patient received their maintenance dose of oxycodone plus either 10 mg, 20 mg, 40 mg or naloxone placebo CR tablets daily (see Table 2).

After the treatment period, patients maintained their maintenance dose of oxycodone only for a further two-week follow-up phase (40 mg, 60 mg, or 80 mg oxycodone per day). Patients maintained a daily diary, and efficacy and safety assessments were performed over the course of the study.

**Table 2: Treatment groups for maintenance phase based on naloxone dose per day.**

| | **Group 1** | | | **Group 2** | | | **Group 3** | | | **Group 4** | | |
|---|---|---|---|---|---|---|---|---|---|---|---|---|
| **Naloxone daily dose (mg)** | Placebo | | | 5+5 | | | 10 + 10 | | | 20 + 20 | | |
| | **0** | | | **10** | | | **20** | | | **40** | | |
| **Oxycodone daily dose (mg)** | 2x20, 2x30, 2x40 | | | 2x20, 2x30, 2x40 | | | 2x20, 2x30, 2x40 | | | 2x20, 2x30, 2x40 | | |
| | **40** | **60** | **80** | **40** | **60** | **80** | **40** | **60** | **80** | **40** | **60** | **80** |
| | | | | | | | | | | | | |
| **Oxycodone + Naloxone dose (mg)** | **40/pl 60/pl 80/pl** | | | **40/10,60/10, 80/10** | | | **40/20, 60/20, 80/20** | | | **40/40, 60/40, 80/40** | | |
| **Ratio** | 40/pl 60/pl 80/pl | | | 4/1, | 6/1, | 8/1 | 2/1, | 3/1, | 4/1 | 1/1, | 1.5/1, | 2/1 |

| | | | | | | | | | | | | |
|---|---|---|---|---|---|---|---|---|---|---|---|---|
| Note: Identical dose ratios were obtained for 40/10 mg and 80/20 mg (4/1) and for 40/20 mg and 80/40 mg (2/1) | | | | | | | | | | | | |

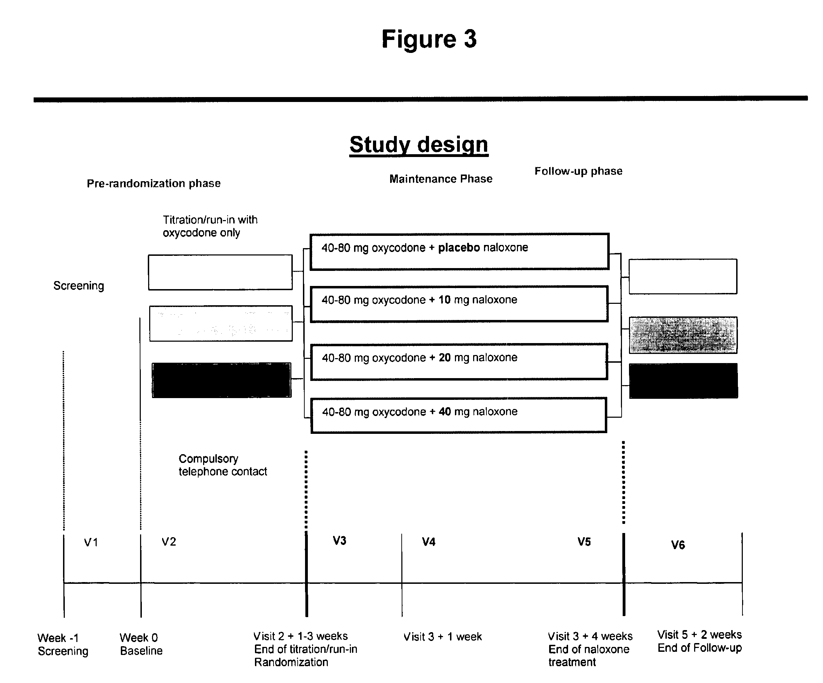

202 subjects were randomized, 196 were in the ITT populations and 166 completed the study. The study design schematic for the clinical study is displayed in Figure 3.

### 3. Primary Efficacy Variables

Efficacy assessments were determined based on data recorded in the case report form and in patient diaries.

The primary efficacy variables of interest were pain and bowel function as follows:
a) Mean Pain during the last 7 days prior to each visit, based on the patient's twice-daily assessment of pain intensity using the 0-100 numerical analogue scale (NAS) (0= no pain and 100= worst imaginable pain). Mean Pain was calculated for each study visit as the mean value of the daily mean values of all patient's diary entries from the last 7 days.
b) Mean bowel function: patient's assessment, at each study visit, of bowel function during the last 7 days prior to each visit. Mean bowel function was calculated from the mean of the three 0-100 NAS scores: ease of defecation (0= easy/no difficulty, 100= severe difficulty), feeling of incomplete bowel evacuation (0= not at all, 100= very strong), and judgment of constipation (0= not at all, 100= very strong).

### 4. Analgesic Efficacy Results

The end of maintenance mean pain results are summarized below:

**Table 3: Mean Pain at End of Titration Visit (V3) and End of Maintenance Visit (V5) by Absolute Dose of Naloxone - ITT (with non-missing data) and PP Analysis Populations.**

| Population | Statistic | Naloxone Placebo | Naloxone 10 mg | Naloxone 20 mg | Naloxone 40 mg |
|---|---|---|---|---|---|
| ITT non- missing | N | 46 | 42 | 43 | 41 |
| | Mean (SD) V3 | 36.9 (15.9) | 35.9 (16.3) | 39.8 (18.4) | 38.1 (15.8) |
| | Mean (SD) V5 | 37.8 (18.2) | 37.2 (17.3) | 37.5 (20.5) | 38.7 (17.0) |
| | 95% Confidence Interval for Difference vs. Placebo* | | (-5.04, 4.58) | (-2.36, 7.22) | (-4.76, 4.93) |
| | | | | | |
| PP | N | 29 | 26 | 22 | 22 |
| | Mean (SD) V3 | 34.0 (16.0) | 38.0 (17.7) | 40.1 (20.0) | 39.0 (16.1) |
| | Mean (SD) V5 | 32.6 (16.6) | 38.8 (18.4) | 36.1 (19.5) | 38.7 (16.6) |
| | 95% Confidence Interval for Difference vs. Placebo* | | (-9.10, 2.94) | (-5.01, 7.64) | (-8.41, 4.22) |

| | | | | | |
|---|---|---|---|---|---|
| *95% Confidence Intervals for Difference vs. Placebo at Visit 5 (end of maintenance) are based on an ANCOVA model with treatment and baseline pain intensity as factors in the model. | | | | | |

The Intent-To-Treat (ITT) population included all randomized patients who received at least one dose of study drug and had at least one post-randomization efficacy assessment. For some analyses, the last observation was carried forward for those ITT subjects who discontinued after Visit 4 (ITT/LOCF). In other instances, only the available data were used (ITT non-missing).

The Per Protocol (PP) population included all randomized patients who completed the study (including the follow-up phase) without major protocol violations.

The differences were small and confidence intervals were fairly narrow relative to the 0-100 pain scale and did not point to a difference in analgesic efficacy between active naloxone and naloxone placebo.

A quadratic response surface model with naloxone and oxycodone dose as factors and baseline pain as covariate shows that the only factor that affects the end of maintenance mean pain is the baseline pain measurement. There was no evidence of changes in mean pain with varying amounts of naloxone. However the study was not designed nor powered as a formal demonstration of non-inferiority of oxycodone/naloxone versus oxycodone/naloxone placebo.

### 5. Bowel Function Efficacy Results

Mean bowel function was calculated for each study visit from the mean of the three NAS values ease/difficulty of defecation, feeling of incomplete bowel evacuation and judgment of constipation. Summary statistics for mean bowel function during the last 7 days were provided for each study visit for the groupings dose ratio of oxycodone and naloxone, absolute dose of naloxone and absolute dose of naloxone given the same oxycodone/naloxone ratio.

To test for difference of absolute dose of naloxone versus placebo, t-tests were performed for the values obtained during the end of maintenance phase (after 4 weeks of naloxone treatment). In addition, two-sided 95% CIs (CI, confidence interval) for the difference in means between the treatment groups were provided. A response surface analysis was also performed for the end of the maintenance phase (after 4 weeks of naloxone treatment). These analyses were performed for the ITT and PP populations. For the ITT population only, t-tests for difference were also performed to explore mean bowel function at Visit 4 (after 1 week of naloxone treatment).

In addition, summary statistics of mean bowel function during the last 7 days for the end of the follow-up phase were provided for the grouping absolute dose of oxycodone in the ITT population.

To evaluate the effects of the titration/run-in period a paired t-test for difference was conducted for the mean bowel function during the last 7 days before the end of titration/run-in, compared with the mean bowel function during the last 7 days before the baseline visit. This analysis was performed in the titration phase population. In addition, two-sided 95% CIs for the difference in means between the treatment periods were provided.

Figures were provided for the ITT and the PP population. The values obtained for mean bowel function during the last 7 days before the end of the maintenance phase (mean ± 95% CI) were plotted against the oxycodone/naloxone dose ratio and the absolute dose of naloxone. In addition, surface plots were provided for the results obtained at the end of the maintenance phase.

To investigate if the bowel function depends on the ratio of oxycodone and naloxone or the absolute dose of naloxone additional analysis and figures were provided for the ITT population. A response surface analysis for the total consumed oxycodone dose during the last week of the maintenance phase versus the naloxone dose was performed. The parameter estimates derived were taken to display a surface plot of the whole dose range investigated. Moreover, a contour plot of the bowel function with a granulation of 10 was performed.

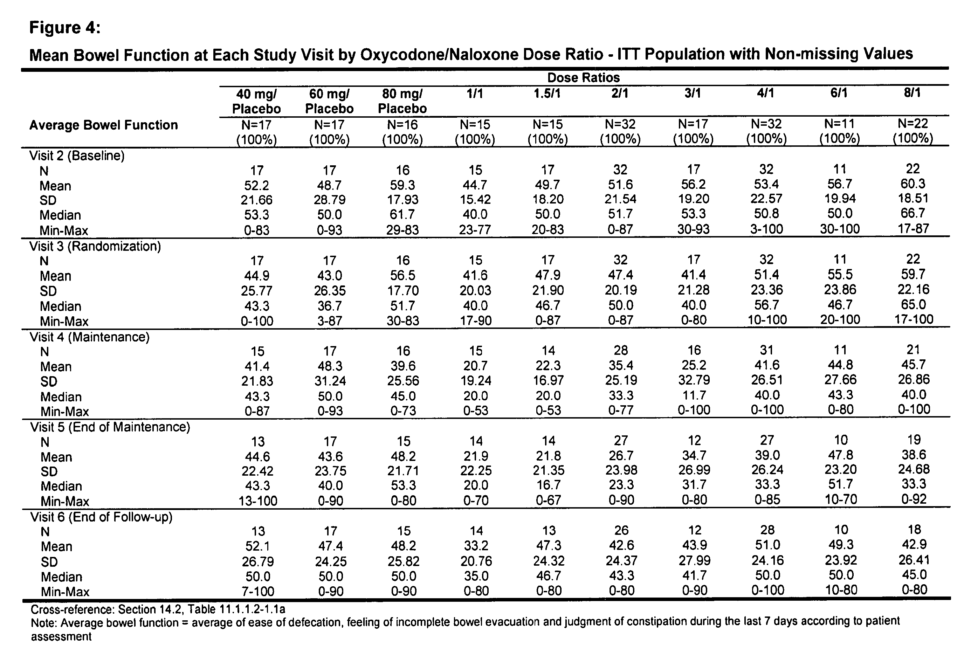

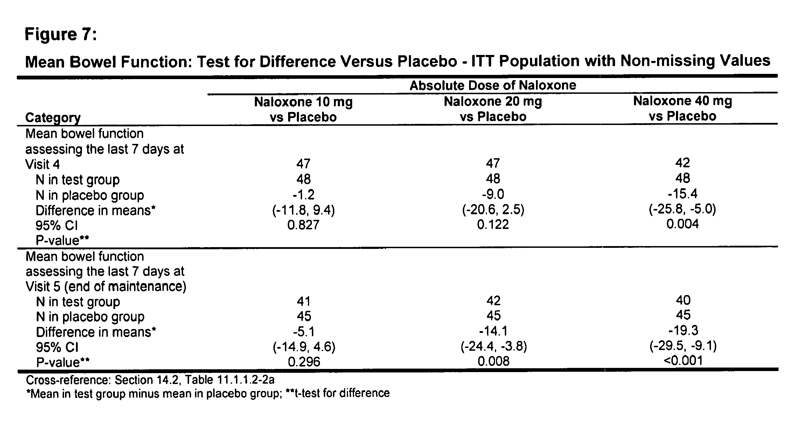

The values for mean bowel function at each study visit by dose ratio, by absolute dose of naloxone and by absolute dose of naloxone given the same oxycodone/naloxone dose ratio in the ITT population are presented in Figures 4 to 6. The test for difference for each dose of naloxone versus placebo is summarized in Figure 7.

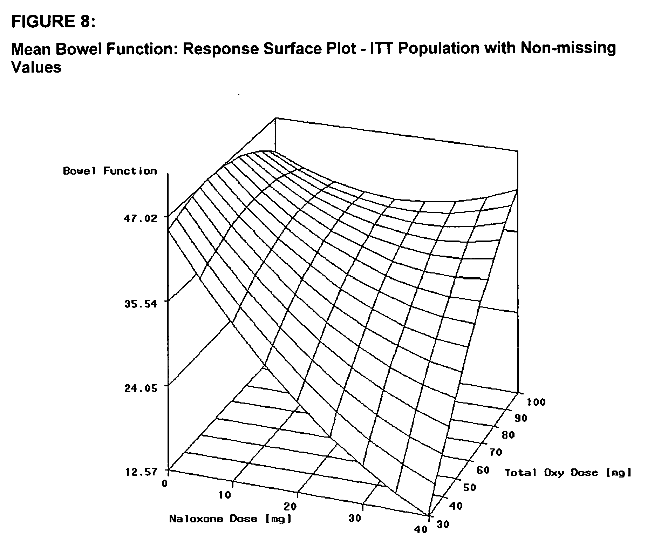

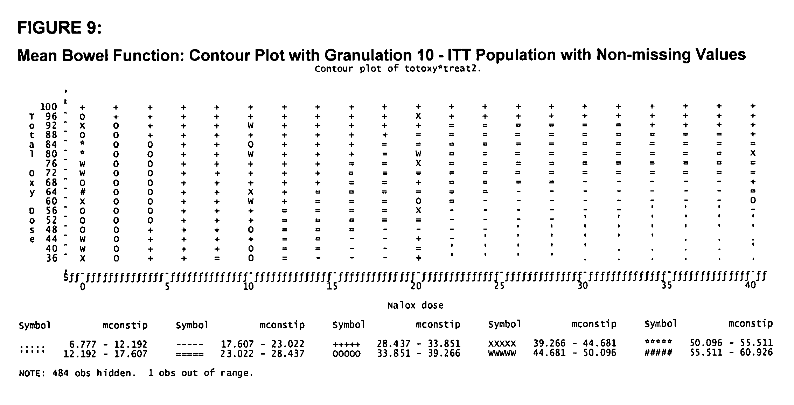

The surface plot of the whole dose range investigated based on the RSREG estimations of the model parameters is displayed in Figure 8. The contour plot of the bowel function with a granulation of 10 is shown in Figure 9.

Within the ITT population, a trend towards improved mean bowel function with increased dose of naloxone was seen. During the last 7 days at the end of the maintenance phase, mean (±SD) bowel function was lowest in the 1/1, 1.5/1 and 2/1 dose ratios (21.9±22.25, 21.8±21.35 and 26.7±23.98 for the 1/1, 1.5/1 and 2/1 dose ratios, respectively). Furthermore, mean bowel function worsened as the amount of naloxone decreased, to a maximum value of 47.8 (±23.20) for a dose ratio of 6/1. For the last 7 days prior to Visit 4, mean bowel function ranged from 20.7 (±19.24) at a ratio of 1/1 to 45.7 (±26.86) at a ratio of 8/1 (see Figure 4. Values for mean bowel function in the oxycodone/naloxone placebo dose ratios were higher than in the 1/1, 1.5/1 and 2/1 dose ratios at both visits.

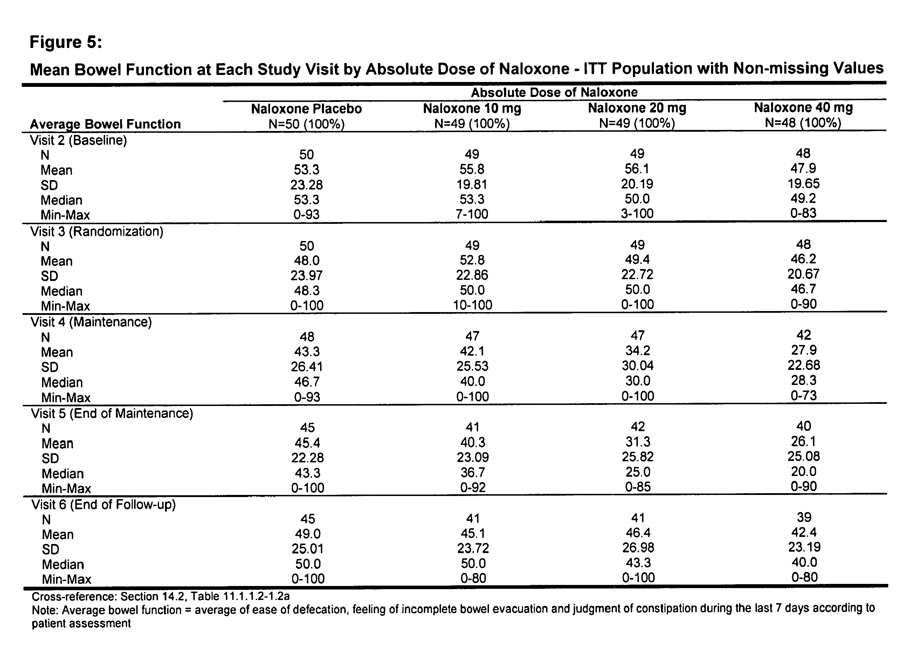

Analysis by absolute dose of naloxone showed values of 45.4 (±22.28), 40.3 (±23.09), 31.3 (±25.82) and 26.1 (±25.08) for placebo, 10 mg, 20 mg and 40 mg respectively at the end of maintenance (p<0.05 for 20 mg and 40 mg naloxone versus placebo, t-test for difference) and 43.3 (±26.41), 42.1 (±25.53), 34.2 (±30.04) and 27.9 (±22.68) at Visit 4 (p=0.004 for 40 mg naloxone versus placebo, t-test for difference) (see Figures 5 and 7).

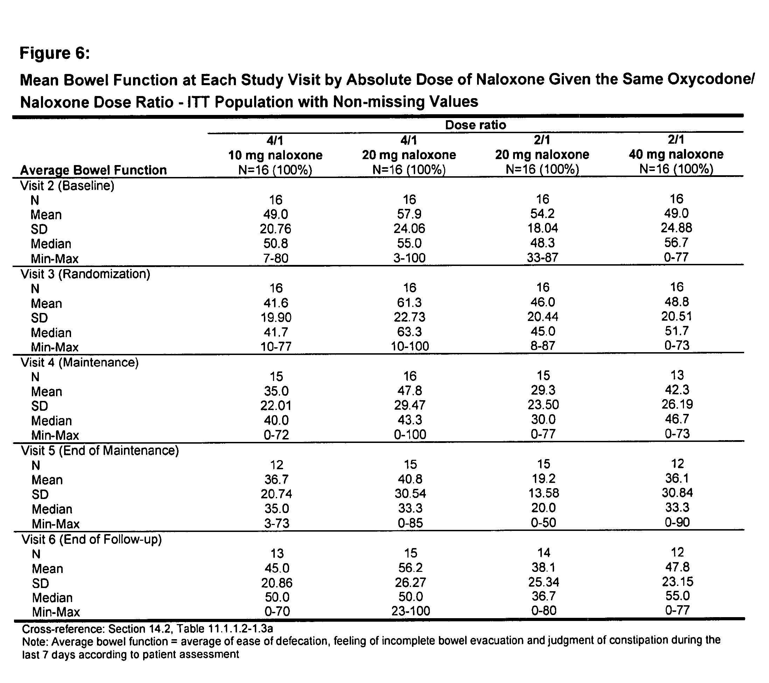

Analysis by absolute dose of naloxone given the same oxycodone/naloxone dose ratio showed that within both dose ratio groups (4/1 and 2/1) patients taking the higher oxycodone dose had higher mean bowel function values at Visits 4 and 5 (see Figure 6).

From the end of the maintenance phase to end of follow-up, mean bowel function worsened (. The range for mean bowel function was 21.8 (±21.35) to 48.2 (±21.71) for the dose ratio groups at end of maintenance and 33.2 (±20.76) to 52.1 (±26.79) for the dose ratio groups at the end of follow-up. The change was greatest in the 40 mg naloxone group; mean bowel function was 26.1 (±25.08) at the end of maintenance and 42.4 (±23.19) at the end of follow-up.

Analysis using the PP population generally mirrored the trends observed in the ITT population with regards to mean bowel function. During the last 7 days at the end of the maintenance phase, mean (±SD) bowel function was lowest in the 1/1 dose ratio (10.7±15.35) and worsened to a maximum of 57.3 (±17.38) for a dose ratio of 6/1. Mean bowel function values were higher than the 1/1, 1.5/1 and 2/1 ratios for all oxycodone/placebo dose ratios. Similar values were seen for the last 7 days prior to Visit 4 with the exception of the 3/1 dose ratio. At the end of the maintenance phase mean bowel function was 42.3 (±24.03), 39.4 (±23.44), 29.8 (±29.29) and 29.6 (±28.34) for placebo, 10 mg, 20 mg and 40 mg naloxone. The small number of patients in each treatment group in the PP population meant statistically significant p-values were not obtained in the PP analysis for t-tests for difference for mean bowel function.

The end of maintenance mean bowel function results are summarized below:

**Table 4: Mean Bowel Function Scores at End of Titration Visit (V3) and End of Maintenance Visit (V5) by Absolute Dose of Naloxone - ITT (non-missing) and ITT/LOCF Analysis Populations.**

| Population | Statistic | Naloxone Placebo | Naloxone 10 mg | Naloxone 20 mg | Naloxone 40 mg |
|---|---|---|---|---|---|
| ITT non-missing | N | 45 | 41 | 42 | 40 |
| | Mean (SD) V3 | 48.2 (23.5) | 53.5 (22.2) | 51.3 (21.6) | 48.2 (20.6) |
| | Mean (SD) V5 | 45.4 (22.3) | 40.3 (23.1) | 31.3 (25.8) | 26.1 (25.1) |
| | P-Value* | | 0.1658 | 0.0025 | 0.0002 |
| | | | | | |
| ITT / LOCF | N | 48 | 47 | 47 | 42 |
| | Mean (SD) V3 | 47.7 (24.0) | 53.6 (22.8) | 49.9 (23.1) | 47.7 (20.5) |
| | Mean (SD) V5 | 44.8 (22.9) | 40.1 (24.7) | 33.2 (28.4) | 26.5 (25.7) |
| | P-Value* | | 0.1795 | 0.0140 | 0.0005 |

| | | | | | |
|---|---|---|---|---|---|
| *Comparison versus Naloxone Placebo using ANCOVA model with Naloxone dose and baseline bowel function as factors in the model. | | | | | |

As already mentioned above, within the ITT population, improved mean bowel function with increased dose of naloxone was seen, with mean values (± SD) of 45.4 (±22.3), 40.3 (±23.1), 31.3 (±25.8) and 26.1 (±25.1) for placebo, 10 mg, 20 mg and 40 mg respectively at the end of maintenance (p<0.05 for 20 mg and 40 mg naloxone versus placebo). The 95% confidence intervals for the mean bowel function differences from naloxone placebo were (-2.83, 16.69) at 10 mg naloxone, (5.46, 24.82) at 20 mg naloxone, and (9.54, 29.11) at 40 mg naloxone. The results display an increasing improvement in bowel function with increasing dose of naloxone, with the difference of the 20mg and 40mg dose versus naloxone placebo statistically significant at end of maintenance.

The response surface quadratic analysis confirms improving bowel function with increasing dose of naloxone, with the linear effect of naloxone dose statistically significant. The Table 5 displays the estimated improvements in mean bowel function scores versus naloxone placebo for the different oxycodone/naloxone ratios studied; these estimates correspond both to oxycodone/naloxone combinations actually represented in the study design, and some combinations for which quadratic surface interpolation was appropriate.

The estimates indicate that the mean bowel function improvement is in general constant within each ratio, and independent of the varying doses of oxycodone and naloxone. The only possible exception is the 80/40 mg combination, where there is a suggestion of a lower predicted effect than for the 60/30 mg and 40/20 mg combinations; this observation, however, has to be interpreted with the size of the standard error in mind.

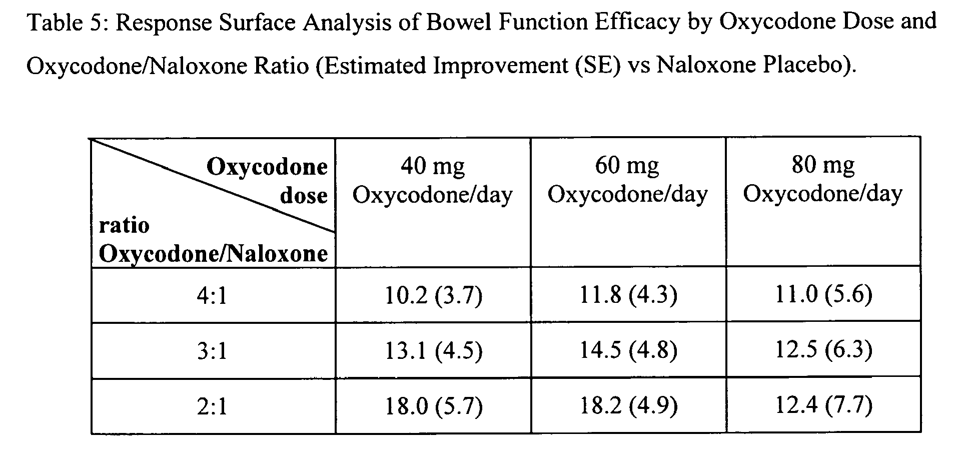

In addition to estimating the treatment effect for individual oxycodone/naloxone combinations, overall treatment effect estimates were obtained for specific ratios. The estimates were calculated by combining the results from the different oxycodone/naloxone combinations, e.g.; the 2:1 ratio estimate was formed by averaging the predicted results of the 40/20 mg, 60/30 mg, and 80/40 mg oxycodone/naloxone combinations, relative to naloxone placebo. The estimated mean differences (SE) in mean bowel function for various oxycodone/naloxone ratios versus naloxone placebo groups are displayed below.

**Table 6: Response Surface Analysis of Bowel Function Efficacy by Oxycodone/Naloxone ratio (Estimated Improvement (SE) vs Naloxone Placebo).**

| **Oxycodone / Naloxone Ratio** | **Overall Improvement (SE) vs Placebo** |
|---|---|
| 6:1 | 8.0 (3.3) |
| 4:1 | 11.1 (4.1) |
| 3:1 | 13.4(4.6) |
| 2:1 | 16.2 (4.5) |
| 1.5:1 | 16.5 (5.1) |

The estimates indicate that bowel function improvement increases as oxycodone/naloxone ratio decreases, with the estimated improvement at 2:1 approximately 50% higher than at 4:1 (p < 0.05) and with a minimal improvement from the 2:1 ratio to the 1.5:1 ratio.

### 6. Study Conclusion

While the study was not designed nor powered as a formal demonstration of non-inferiority of oxycodone/naloxone versus oxycodone/naloxone placebo, the administration of prolonged oxycodone and naloxone in combination was not associated descriptively with differences in the intensity of mean pain whether analyzed by dose ratios or absolute dose of naloxone.

The study demonstrated that addition of controlled release naloxone to controlled release oxycodone results in a statistically significant improvement in mean bowel function at the two higher doses of naloxone (20mg and 40mg). The improvement increases with decreasing oxycodone/naloxone ratio and appears to plateau at the 2:1 ratio, with the overall effect at 2:1 ratio approximately 50% greater than at 4:1. The data indicate that the bowel function improvement is in general a function of the ratio; i.e., the improvement is, in general, constant within each ratio, and independent of the varying doses of oxycodone and naloxone. The only exception is the 80/40 combination, where there is a suggestion of a lower predicted effect than for the 60/30 mg and 40/20 mg combinations; this observation, however, has to be interpreted with the size of the standard error in mind.

### Example 2: Pharmacokinetic and bioavailability characteristics of different strengths of a fixed combination of oxycodone and naloxone and a combination of Oxygesic® plus Naloxone CR

A melt extrusion oxycodone/naloxone controlled-release tablet formulation with an oxycodone:naloxone ratio of 2:1 has been produced. There are three dose strengths available, namely OXN 10/5, OXN 20/10, and OXN 40/20, where the first number is the mg amount of oxycodone hydrochloride and the second number is the mg amount of naloxone hydrochloride (see Table 7). OXN 20/10 and OXN 40/20 are from the same granulate, while OXN 10/5 has a slightly different formula in regard to the ratio of active ingredients to excipients.

This study investigated the pharmacokinetics of all dose strengths of the new combination product and compared its bioavailability relative to that of oxycodone in the marketed form, Oxygesic®, given together with an experimental controlled-release naloxone tablet.

The objectives of this study were to (i) evaluate the pharmacokinetic and bioavailability parameters of oxycodone and naloxone and their main metabolites when administered as a controlled-release fixed combination tablet formulation; (ii) assess the interchangeability between the 3 different strengths of the fixed combination, OXN 10/5, OXN 20/10 and OXN 40/20; and (iii) compare the pharmacokinetics and bioavailability of the fixed combination formulation with marketed Oxygesic® given together with Naloxone CR tablets;

### 1. Pharmaceutics

Oxycodone/naloxone tablets (OXN Tablets) according to this example contain a fixed combination of oxycodone and naloxone in the ratio of 2:1. Tablets formulations are summarized below (see Table 7).

The 20/10 mg and 40/20 mg tablets will be manufactured from the same granulation with these two tablet strengths being compositionally proportional. Oxycodone/Naloxone prolonged release tablets (OXN) tablets according to this example are controlled release tablets using a matrix of stearyl alcohol and ethylcellulose as the retardant. The tablets contain the combination of oxycodone hydrochloride and naloxone hydrochloride in the strengths 10/5 mg, 20/10 mg and 40/20 mg (both as the hydrochloride). The complete statement of the components and quantitative composition of Oxycodone/Naloxone prolonged release tablets is given below in Table 7.

**Table 7: Oxycodone/Naloxone prolonged release tablets.**

| **Component** | **Quantity (mg/tablet)** | | | **Function** | **Reference to Standard** |
|---|---|---|---|---|---|
| | OXN 10/5 | OXN 20/10 | OXN 40/20 | | |
| Oxycodone hydrochloride 1) | 10.50 | 21.00 | 42.00 | Active | USP*/ H.S.E. |
| *corresponding to* | | | | | |
| *Oxycodone hydrochloride anhydrous* | *10.00* | *20.00* | *40.00* | | |
| *Oxycodone base* | *9.00* | *18. 00* | *36.00* | | |
| Naloxone hydrochloride Dihydrate | 5.45 | 10.90 | 21.80 | Active | Ph. Eur.* |
| *corresponding to* | | | | | |
| *Naloxone hydrochloride anhydrous* | *5.00* | *10.00* | *20.00* | | |
| *Naloxone base* | *4.50* | *9.00* | *18.00* | | |
| Povidone K30 | 5.00 | 7.25 | 14.50 | Binder | Ph. Eur.* |
| Ethylcellulose N 45 | 10.00 | 12.00 | 24.00 | Retardant | Ph. Eur.* |
| Stearyl alcohol | 25.00 | 29.50 | 59.00 | Retardant | Ph. Eur.* |
| Lactose monohydrate | 64.25 | 54.50 | 109.00 | Diluent | Ph. Eur.* |
| Purified talc | 2.50 | 2.50 | 5.00 | Glidant | Ph. Eur.* |
| Magnesium stearate | 1.25 | 1.25 | 2.50 | Lubricant | Ph. Eur.* |
| **Total core** | **123.95** | **138.90** | **277.80** | | |
| **Film Coat °** | | | | | |
| Opadry II HP white - 85F18422 | 3.72 | | | Coating | supplier specification |
| Opadry II HP pink - 85F24151 | | 4.17 | | Coating | supplier specification |
| Opadry II HP yellow 85F32109 | | | 8.33 | Coating | supplier specification |
| Purified talc | 0.12 | 0.14 | 0.28 | Gloss | Ph. Eur.* |
| **Total filmtablet** | **127,79** | **143.21** | **286.41** | | *** current** Edition |

| | | | | | |
|---|---|---|---|---|---|
| ¹⁾ calculated based on expected moisture content | | | | | |
| ° qualitative composition : see Table 8 | | | | | |

**Table 8: Qualitative composition of the film coat.**

| **Opadry II HP** | **white 85F18422** | **pink 85F24151** | **yellow 85F32109** | **Reference to Standard** |
|---|---|---|---|---|
| Polyvinylalcohol part. hydrolized | + | + | + | Ph. Eur. * |
| Titanium dioxide (E 171) | + | + | + | Ph. Eur. * |
| Macrogol 3350 | + | + | + | Ph. Eur. * |
| Talcum | + | + | + | Ph. Eur. * |
| Iron oxide red (E 172) | | + | | NF* /EC Directive |
| Iron oxide yellow (E 172) | | | + | NF* /EC Directive |
| | | | | * current Edition |

### 2. Pharmacokinetic Assessments

### a) Drug Concentration Measurements

Blood samples for determining oxycodone, noroxycodone, oxymorphone, noroxymorphone, naloxone, 6β-naloxol and naloxone-3-glucuronide concentrations were obtained for each subject during each of the 4 study periods immediately before dosing; and at 0.5, 1, 1.5, 2, 2.5, 3, 3.5, 4, 5, 6, 8, 10, 12, 16, 24, 28, 32, 36, 48, 72 and 96 hours (22 blood samples per study period) after dosing. Blood was also drawn where possible at the first report of a serious or severe unexpected adverse event and at its resolution.

At each time of plasma determination, 6 mL venous blood was drawn from a forearm vein into a tube containing K2 EDTA anticoagulant. All samples were processed according to common sample handling procedures.

### b) Pharmacokinetic Parameters

The following pharmacokinetic parameters were calculated from the plasma concentrations of oxycodone, noroxycodone, oxymorphone, noroxymorphone, naloxone, 6β-naloxol and naloxone-3-glucuronide:
- Area under the plasma concentration time curve calculated to the last measurable concentration (AUCt);
- Area under the plasma concentration-time curve, from the time of administration to infinity (AUCINF);
- Maximum observed plasma concentration (Cₘₐₓ);
- Time point of maximum observed plasma concentration (tₘₐₓ);
- Terminal phase rate constant (LambdaZ);
- Apparent terminal phase half life (t½Z).
   For oxycodone, noroxycodone, oxymorphone, noroxymorphone, and naloxone-3-glucuronide, AUC values were given in ng·h/mL, and Cₘₐₓ values in ng/mL. For naloxone and 6β-naloxol, AUC values, due to the low concentrations, were given in pg·h/mL and Cₘₐₓ values in pg/mL.
   AUCt, AUCINF and Cₘₐₓ were regarded as the primary parameters.
   AUCt were calculated using the linear trapezoidal method. Where possible, LambdaZ was estimated using those points determined to be in the terminal log-linear phase. t½Z was determined from the ratio of In 2 to LambdaZ. The areas under the plasma concentration-time curve between the last measured point and infinity were calculated from the ratio of the final observed plasma concentration (Cₗₐₛₜ) to LambdaZ. This was then added to the AUCt to yield AUCINF.
   All pharmacokinetic calculations were performed with WinNonlin Enterprise Edition, Version 4.1.

### 3. Statistical Methods

Cₘₐₓ and AUCINF of oxycodone were important in order to assess the equivalence of the 4 treatments. AUCt was calculated using the linear trapezoidal method. Where possible, LambdaZ was estimated using those points determined to be in the terminal log-linear phase. t½Z were determined from the ratio of In 2 to LambdaZ. The areas under the plasma concentration-time curve between the last measured point and infinity were calculated from the ratio of the final observed plasma concentration (Cₗₐₛₜ) to LambdaZ. This was added to the AUCt to yield the area under the plasma concentration-time curve between the time of administration and infinity (AUCINF).

The dose adjusted relative systemic availabilities (Frelt, and FrelINF) and the Cₘₐₓ ratio were obtained from the ratio of AUCt, AUCINF and Cₘₐₓ values, respectively, for differences defined in the following comparisons of interest:
fixed combination A vs. open combination D
fixed combination B vs. open combination D
fixed combination C vs. open combination D
fixed combination A vs. fixed combination B
fixed combination A vs. fixed combination C
fixed combination B vs. fixed combination C

The full analysis population for pharmacokinetics were used for these analyses.

The metabolite: parent drug AUCt and AUCINF ratios were estimated for each treatment, where possible.

All safety data were evaluated by descriptive methods only.

### 4. Clinical Pharmacology Results

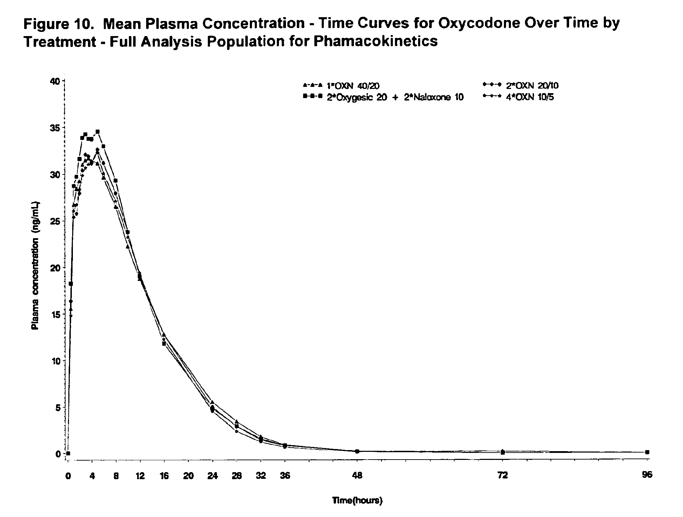

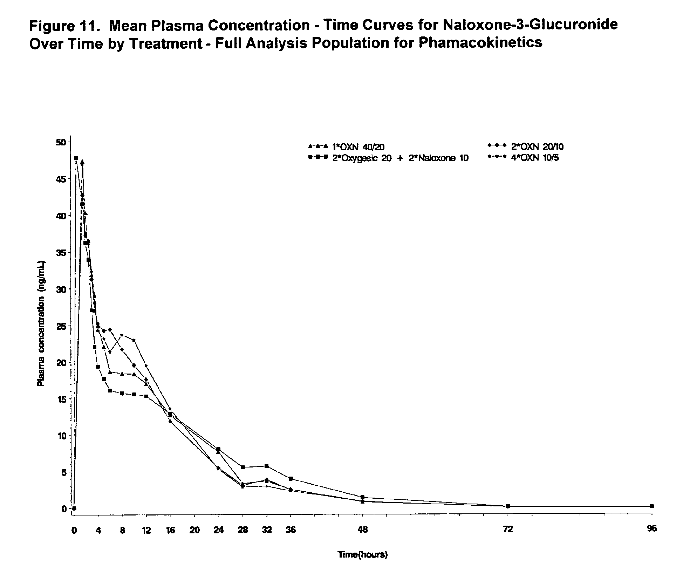

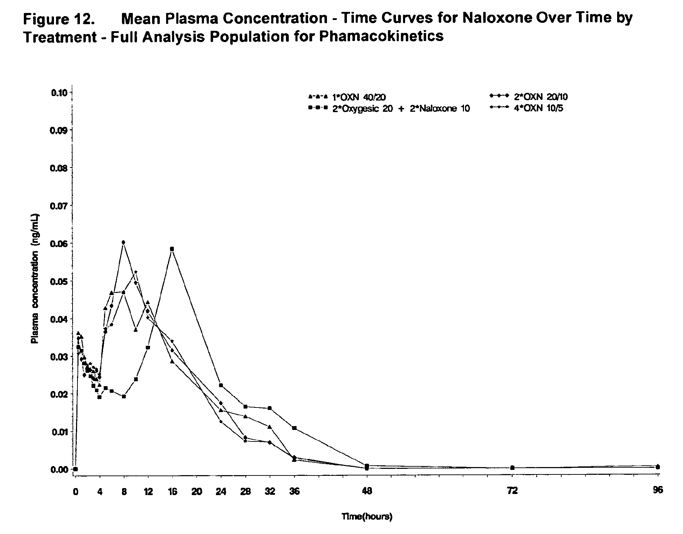

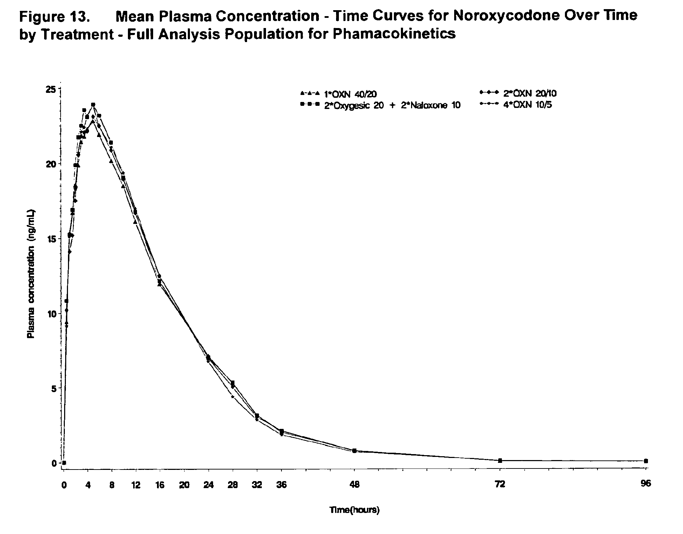

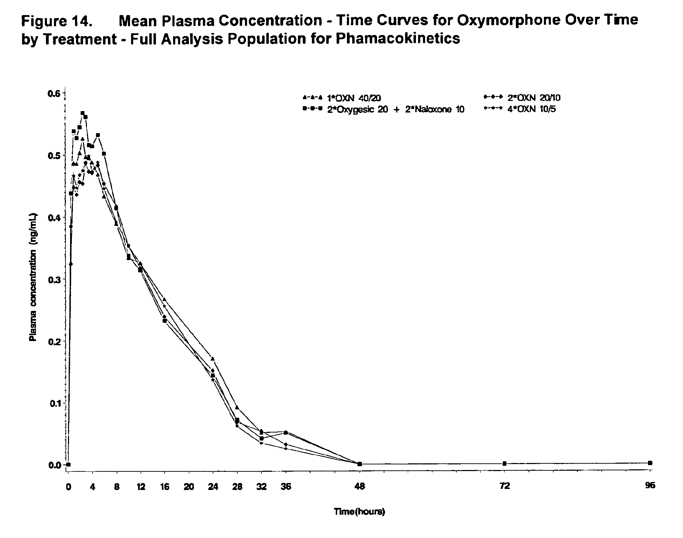

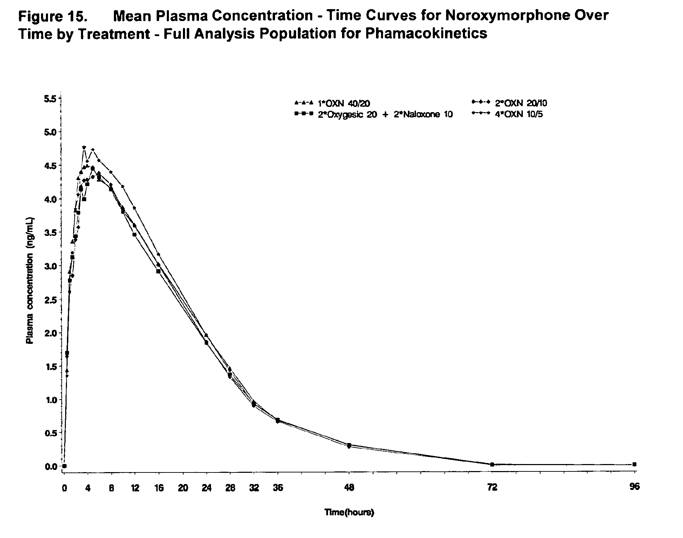

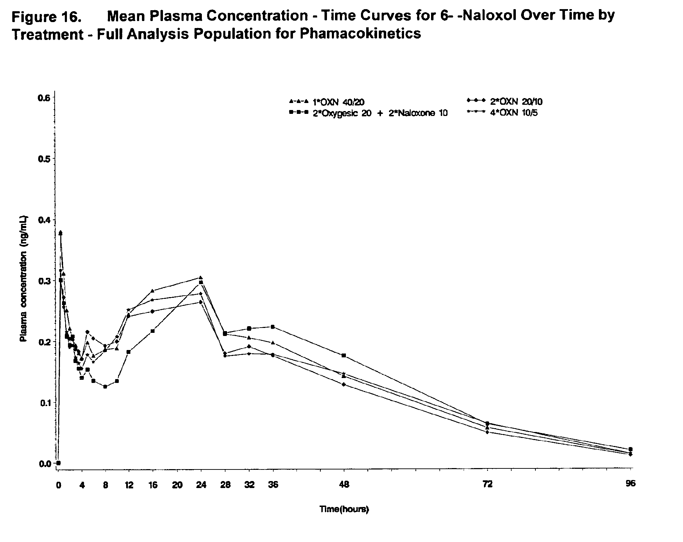

Mean observed plasma concentration - time curves for oxycodone, naloxone-3-glucuronide, naloxone, noroxycodone, oxymorphone, noroxymorphone and 6-β-naloxol are presented in Figures 10 to 16.

Pharmacokinetic parameters for oxycodone, naloxone-3-glucuronide and naloxone are presented in Tables 9 to 14 respectively.

**Table 9: Summary of Pharmacokinetic Parameters for Oxycodone by Treatment: Full Analysis Population for Pharmacokinetics**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 24 | 23 | 23 | 23 |
| Arithmetic Mean | 473.49 | 491.22 | 488.89 | 502.28 |
| (SD) | (72.160) | (82.181) | (91.040) | (84.128) |
| Geometric Mean | 468.29 | 484.58 | 481.08 | 495.72 |
| **AUCINF (ng.h/mL)** | | | | |
| N | 24 | 22 | 22 | 22 |
| Arithmetic Mean | 475.06 | 497.17 | 491.22 | 509.11 |
| (SD) | (72.182) | (81.687) | (93.458) | (82.963) |
| Geometric Mean | 469.87 | 490.65 | 483.04 | 502.80 |
| **Cmax (ng/mL)** | | | | |
| N | 24 | 23 | 23 | 23 |
| Arithmetic Mean | 34.91 | 35.73 | 34.46 | 40.45 |
| (SD) | (4.361) | (4.931) | (5.025) | (4.706) |
| Geometric Mean | 34.66 | 35.41 | 34.12 | 40.19 |
| **tmax (h)** | | | | |
| N | 24 | 23 | 23 | 23 |
| Median | 3.5 | 4.0 | 3.0 | 2.5 |
| (Min,Max) | (1.0,6.0) | (2.0,8.0) | (1.0,6.0) | (0.5,8.0) |
| **t1/2Z** | | | | |
| N | 24 | 22 | 22 | 22 |
| Arithmetic Mean | 4.69 | 4.87 | 4.83 | 5.01 |
| (SD) | (0.775) | (0.995) | (0.975) | (0.802) |

**Table 10. Oxycodone Summary of Ratios for AUCt, AUCINF, Cₘₐₓ and Differences for tₘₐₓ and Half-Life - Full Analysis Population for Pharmacokinetics.**

| **Pharmacokinetic metric** | **4 x OXN 10/5/2 x Oxygesic 20 + 2 x Naloxone 10** | **2 x OXN 20/10/2 x Oxygesic 20 + 2 x Naloxone 10** | **1 x OXN 40/20/2 x Oxygesic 20 + 2 x Naloxone 10** | **4 x OXN 10/5/ 2 x OXN 20/10** | **4 x OXN 10/5 /1xOXN 40/20** | **2 x OXN 20/10/ 1 x OXN 40/20** |
|---|---|---|---|---|---|---|
| **AUCt (ng.h/mL)** | | | | | | |
| Ratio (%) | 94.9 | 98.2 | 98.0 | 96.7 | 96.8 | 100.2 |
| 90%CI | 91.5,98.5 | 94.5, 102.0 | 94.4, 101.7 | 93.1, 100.4 | 93.3, 100.5 | 96.5, 104.0 |
| **AUCINF(ng.h/mL)** | | | | | | |
| Ratio (%) | 94.5 | 98.2 | 97.8 | 96.2 | 96.5 | 100.4 |
| 90%CI | 90.9, 98.1 | 94.5,102.1 | 94.1,101.7 | 92.6,99.9 | 92.9,100.3 | 96.5,104.3 |
| **Cmax (ng/mL)** | | | | | | |
| Ratio (%) | 86.2 | 88.4 | 85.8 | 97.5 | 100.5 | 103.1 |
| 90%CI | 82.2,90.4 | 84.2, 92.8 | 81.8, 90.0 | 92.9, 102.3 | 95.8, 105.4 | 98.2, 108.1 |
| **tmax (h)** | | | | | | |
| Difference | 0.49 | 1.11 | 0.14 | -0.63 | 0.35 | 0.97 |
| 90%CI | -0.19, 1.16 | 0.42, 1.80 | -0.54,0.82 | -1.31,0.05 | -0.33, 1.02 | 0.29,1.66 |
| **t1/2Z (h)** | | | | | | |
| Difference | -0.27 | -0.11 | -0.11 | -0.16 | -0.16 | 0.00 |
| 90%CI | -0.60, 0.05 | -0.44, 0.23 | -0.44, 0.22 | -0.49,0.16 | -0.49, 0.16 | -0.33, 0.33 |

**Table 11. Summary of Pharmacokinetic Parameters for Naloxone-3-glucuronide by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (pg.h/mL)** | | | | |
| N | 24 | 23 | 23 | 23 |
| Arithmetic Mean (SD) | 539.93 | 522.45 | 520.10 | 523.37 |
| Geometric Mean | (142.241) | (128.569) | (133.175) | (119.752) |
| | 520.14 | 506.63 | 502.26 | 509.38 |
| **AUCINF(pg.h/mL)** | | | | |
| N | 22 | 21 | 22 | 22 |
| Arithmetic Mean (SD) | 562.53 | 520.97 | 527.94 | 537.25 |
| Geometric Mean | (130.732) | (133.172) | (135.424) | 110.829 |
| | 546.73 | 504.34 | 509.62 | 525.91 |
| **Cmax (pg/mL)** | | | | |
| N | 24 | 23 | 23 | 23 |
| Arithmetic Mean (SD) | 62.01 | 63.62 | 61.95 | 63.55 |
| Geometric Mean | (15.961) | (19.511) | (18.369) | (16.748) |
| | 59.93 | 60.70 | 59.34 | 61.55 |
| **tmax (h)** | | | | |
| N | 24 | 23 | 23 | 23 |
| Median | 1.0 | 0.5 | 1.0 | 1.0 |
| (Min,Max) | (0.5, 3.0) | (0.5, 6.0) | (0.5,3.0) | (0.5, 6.0) |
| **t1/2Z** | | | | |
| N | 22 | 21 | 22 | 22 |
| Arithmetic Mean (SD) | 8.48 | 7.93 | 7.81 | 7.66 |
| | (3.066) | (2.402) | (2.742) | (1.717) |

**Table 12. Naloxone-3-Glucuronide Summary of Ratios for AUCt, AUCINF, Cₘₐₓ and Differences for Tₘₐₓ and Half-Life - Full Analysis Population for Pharmacokinetics.**

| **Pharmacokinetic metric** | **4 x OXN 10/5 / 2 x Oxygesic 20 + 2 x Naloxone 10** | **2 x OXN 20/10 / 2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5 /1 x OXN 40/20** | **2 x OXN 20/10 / 1 x OXN 40/20** |
|---|---|---|---|---|---|---|
| **AUCt (pg.h/mL)** | | | | | | |
| Ratio (%) | 101.0 | 98.8 | 98.6 | 102.2 | 102.4 | 100.2 |
| 90%CI | 95.6, 106.8 | 93.4, 104.5 | 93.3, 104.3 | 96.7, 108.1 | 97.0, 108.2 | 94.8, 105.9 |
| **AUCINF(pg.h/mL)** | | | | | | |
| Ratio (%) | 102.1 | 98.2 | 99.0 | 104.0 | 103.1 | 99.2 |
| 90%Cl | 96.3, 108.3 | 92.3, 104.2 | 93.4, 105.0 | 97.9, 110.5 | 97.3, 109.3 | 93.5, 105.2 |
| **Cmax (pg/mL)** | | | | | | |
| Ratio (%) | 95.4 | 96.5 | 95.1 | 98.8 | 100.3 | 101.5 |
| 90%Cl | 88.5, 102.8 | 89.4, 104.1 | 88.2, 102.5 | 91.7, 106.6 | 93.1, 108.0 | 94.1, 109.3 |
| **tmax (h)** | | | | | | |
| Difference | -0.34 | -0.16 | -0.42 | -0.18 | 0.08 | 0.26 |
| 90%CI | -0.84,0.17 | -0.67,0.35 | -0.93,0.10 | -0.69,0.33 | -0.43,0.59 | -0.26,0.77 |
| **t1/2Z (h)** | | | | | | |
| Difference | 0.87 | 0.37 | 0.32 | 0.50 | 0.56 | 0.06 |
| 90%CI | -0.02, 1.77 | -0.53, 1.28 | -0.58, 1.21 | -0.41, 1.41 | -0.33, 1.45 | -0.85, 0.96 |

**Table 13. Summary of Pharmacokinetic Parameters for Naloxone by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (pg.h/mL)** | | | | |
| N | 24 | 23 | 23 | 23 |
| Arithmetic Mean | 0.84 | 0.89 | 0.87 | 0.97 |
| (SD) | (0.656) | (0.749) | (0.718) | (0.976) |
| Geometric Mean | 0.67 | 0.70 | 0.68 | 0.72 |
| **AUCINF(pg.h/mL)** | | | | |
| N | 2 6 0 | | | 1 |
| Arithmetic Mean | - | 1.64 | - | - |
| (SD) | - | (1.043) | - | - |
| Geometric Mean | - | 1.45 | - | - |
| **Cmax (pg/mL)** | | | | |
| N | 24 | 23 | 23 | 23 |
| Arithmetic Mean | 0.07 | 0.08 | 0.08 | 0.08 |
| (SD) | (0.065) | (0.106) | (0.071) | (0.101) |
| Geometric Mean | 0.06 | 0.06 | 0.06 | 0.06 |
| **tmax (h)** | | | | |
| N | 24 | 23 | 23 | 23 |
| Median | 4.0 | 5.0 | 2.0 | 1.0 |
| (Min,Max) | (0.5, 12.0) | (0.5,24.0) | (0.5, 12.0) | (0.5,24.0) |
| **t1/2Z** | | | | |
| N | 4 | 9 | 4 | 4 |
| Arithmetic Mean | 9.89 | 12.85 | 13.83 | 11.02 |
| (SD) | (3.137) | (11.924) | (1.879) | (1.075) |

**Table 14. Naloxone Summary of Ratios for AUCt, AUCINF, Cₘₐₓ and Differences for Tₘₐₓ and Half-Life - Full Analysis Population for Pharmacokinetics.**

| **Pharmacokin etic metric** | **4 x OXN 10/5 / 2 x Oxygesic 20 + 2 x Naloxone 10** | **2 x OXN 20/10 / 2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5 /1 x OXN 40/20** | **2 x OXN 20/10 / 1 x OXN 40/20** |
|---|---|---|---|---|---|---|
| **AUCt(pg.h/mL)** | | | | | | |
| Ratio (%) | 94.2 | 99.4 | 94.1 | 94.7 | 100.1 | 105.7 |
| 90%CI | 82.0, 108.2 | 86.3, 114.5 | 81.8, 108.1 | 82.4, 108.9 | 87.3, 114.9 | 92.0, 121.5 |
| **AUCINF (pg.h/mL)** | | | | | | |
| Ratio (%) | - | - | - | - | - | - |
| 90%CI | -- | -- | -- | -- | -- | -- |
| **Cmax (pg/mL)** | | | | | | |
| Ratio (%) | 102.4 | 108.8 | 104.1 | 94.1 | 98.4 | 104.5 |
| 90%CI | 88.0, 119.2 | 93.1, 127.0 | 89.3, 121.2 | 80.8, 109.7 | 84.6, 114.4 | 89.7, 121.8 |
| **tmax (h)** | | | | | | |
| Difference | -0.71 | 0.12 | -2.03 | -0.83 | 1.32 | 2.15 |
| 90%CI | -2.96, 1.54 | -2.17, 2.42 | -4.31, 0.24 | -3.10, 1.44 | -0.93, 3.57 | -0.12,4.43 |
| **t1/2Z (h)** | | | | | | |
| Difference | -3.55 | 0.79 | 2.30 | -4.35 | -5.85 | -1.51 |
| 90%CI | -12.92, 5.82 | -23.09, 24.67 | -22.06,26.67 | -28.49, 19.80 | -30.48, 18.77 | -8.80,5.78 |

**Table 15. Summary of Pharmacokinetic Parameters for Noroxycodone by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 23 | 23 | 23 | 23 |
| Arithmetic Mean | 439.71 | 442.70 | 436.15 | 451.35 |
| (SD) | (194.093) | (208.868) | (192.795) | (219.059) |
| Geometric Mean | 405.22 | 403.63 | 401.90 | 408.91 |
| **AUCINF (ng.h/mL)** | | | | |
| N | 23 | 22 | 22 | 22 |
| Arithmetic Mean | 447.28 | 453.05 | 440.75 | 462.53 |
| (SD) | (197.697) | (210.830) | (197.780) | (221.201) |
| Geometric Mean | 411.57 | 413.50 | 404.89 | 419.45 |
| **Cmax (ng/mL)** | | | | |
| N | 24 | 23 | 23 | 23 |
| Arithmetic Mean | 24.69 | 25.55 | 24.26 | 26.67 |
| (SD) | (6.507) | (6.986) | (6.415) | (8.428) |
| Geometric Mean | 23.83 | 24.56 | 23.42 | 25.38 |
| **tmax (h)** | | | | |
| N | 24 | 23 | 23 | 23 |
| Median | 5.0 | 5.0 | 3.5 | 4.0 |
| (Min,Max) | (2.0,8.0) | (2.5,8.0) | (2.0,8.0) | (1.0,8.0) |
| **t1/2Z (h**^{**-1**}**)** | | | | |
| N | 23 | 22 | 22 | 22 |
| Arithmetic Mean | 7.03 | 7.10 | 7.25 | 6.95 |
| (SD) | (1.679) | (1.598) | (1.587) | (1.539) |
| **Noroxycodone:oxycodone** | | | | |
| **AUCt ratio (ng.h/mL)** | | | | |
| N | 24 | 23 | 23 | 23 |
| Arithmetic Mean | 0.93 | 0.91 | 0.91 | 0.91 |
| (SD) | (0.368) | (0.393) | (0.404) | (0.444) |
| **Noroxycodone:oxycodone** | | | | |
| **AUCINF ratio (ng.h/mL)** | | | | |
| N | 23 | 21 | 21 | 22 |
| Arithmetic Mean | 0.94 | 0.92 | 0.90 | 0.92 |
| (SD) | (0.374) | (0.408) | (0.420) | (0.449) |

**Table 16. Noroxycodone Summary of Ratios for AUCt, AUCINF, Cₘₐₓ and Differences for Tₘₐₓ and Half-Life - Full Analysis Population for Pharmacokinetics.**

| **Pharmacokinetic metric** | **4 x OXN 10/5/2 x Oxygesic 20 + 2 x Naloxone 10** | **2 x OXN 20/10/2 x Oxygesic 20 + 2 x Naloxone 10** | **1 x OXN 40/20/2 x Oxygesic 20 + 2 x Naloxone 10** | **4 x OXN 10/5/ 2 x OXN 20/10** | **4 x OXN 10/5 /1 x OXN 40/20** | **2 x OXN 20/10/ 1 x OXN 40/20** |
|---|---|---|---|---|---|---|
| **AUCt (ng.h/mL)** | | | | | | |
| Ratio (%) | 98.0 | 97.2 | 97.7 | 100.8 | 100.3 | 99.5 |
| 90%CI | 95.3, 100.8 | 94.4, 100.1 | 95.0, 100.5 | 98.0, 103.7 | 97.5, 103.2 | 96.7, 102.4 |
| **AUCINF(ng.h/mL)** | | | | | | |
| Ratio (%) | 97.2 | 97.3 | 97.7 | 99.8 | 99.5 | 99.6 |
| 90%Cl | 94.4, 100.0 | 94.5, 100.3 | 94.9, 100.6 | 97.0, 102.8 | 96.7, 102.3 | 96.8, 102.6 |
| **Cmax (ng/mL)** | | | | | | |
| Ratio (%) | 91.7 | 94.5 | 90.4 | 97.0 | 101.4 | 104.5 |
| 90%CI | 87.7,95.8 | 90.4,98.8 | 86.5,94.5 | 92.8, 101.4 | 97.1, 105.9 | 100.0, 109.2 |
| **tmax (h)** | | | | | | |
| Difference | 0.18 | 0.30 | 0.20 | -0.12 | -0.02 | 0.10 |
| 90%CI | -0.47,0.84 | -0.37,0.97 | -0.46,0.86 | -0.78,0.54 | -0.67,0.64 | -0.56,0.76 |
| **t1/2Z (h)** | | | | | | |
| Difference | 0.13 | 0.25 | 0.33 | -0.12 | -0.20 | -0.08 |
| 90%CI | -0.20,0.46 | -0.09,0.59 | -0.00,0.66 | -0.45,0.21 | -0.53,0.12 | -0.41,0.25 |

**Table 17. Summary of Pharmacokinetic Parameters for Oxymorphone by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 24 | 23 | 23 | 23 |
| Arithmetic Mean | 8.08 | 8.30 | 8.72 | 8.61 |
| (SD) | (4.028) | (4.276) | (4.586) | (4.463) |
| Geometric Mean | 6.81 | 6.11 | 6.73 | 6.95 |
| **AUCINF (ng.h/mL)** | | | | |
| N | 4 | 5 | 4 | 6 |
| Arithmetic Mean | 13.73 | 12.69 | 17.69 | 11.28 |
| (SD) | (3.538) | (4.176) | (3.200) | (4.400) |
| Geometric Mean | 13.37 | 12.09 | 17.48 | 10.48 |
| **Cmax (ng/mL)** | | | | |
| N | 24 | 23 | 23 | 23 |
| **Arithmetic Mean** | 0.57 | 0.58 | 0.61 | 0.72 |
| (SD) | (0.223) | (0.248) | (0.234) | (0.328) |
| Geometric Mean | 0.53 | 0.52 | 0.56 | 0.63 |
| **tmax (h)** | | | | |
| N | 24 | 23 | 23 | 23 |
| Median | 2.0 | 2.0 | 2.0 | 2.0 |
| (Min,Max) | (0.5, 6.0) | (0.5, 8.0) | (0.5, 4.0) | (0.5, 6.0) |
| **t1/2Z (h**^{**-1**}**)** | | | | |
| N | 14 | 9 | 13 | 12 |
| Arithmetic Mean | 11.06 | 10.66 | 14.09 | 12.14 |
| (SD) | (3.261) | (1.766) | (8.540) | (4.803) |
| **Oxymorphone:oxycodone** | | | | |
| **AUCt ratio(ng.h/mL)** | | | | |
| N | 24 | 23 | 23 | 23 |
| Arithmetic Mean | 0.02 | 0.02 | 0.02 | 0.02 |
| (SD) | (0.009) | (0.009) | (0.010) | (0.011) |
| **Oxymorphone:oxycodone** | | | | |
| **AUCINF ratio(ng.h/mL)** | | | | |
| N | 4 5 4 | | | 5 |
| Arithmetic Mean | 0.03 | 0.02 | 0.03 | 0.03 |
| (SD) | (0.006) | (0.008) | (0.012) | (0.011) |

**Table 18. Oxymorphone Summary of Ratios for AUCt, AUCINF, Cₘₐₓ and Differences for Tₘₐₓ and Half-Life - Full Analysis Population for Pharmacokinetics.**

| **Pharmacokinetic metric** | **4 x OXN 10/5 / 2 x Oxygesic 20 + 2 x Naloxone 10** | **2 x OXN 20/10**/**2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5 / 1 x OXN 40/20** | **2 x OXN 20/10 / 1 x OXN 40/20** |
|---|---|---|---|---|---|---|
| **AUCt (ng.h/mL)** | | | | | | |
| Ratio (%) | 98.2 | 89.9 | 97.4 | 109.3 | 100.8 | 92.2 |
| 90%CI | 82.4,117.0 | 75.1, 107.5 | 81.7, 116.2 | 91.6,130.4 | 84.7,119.9 | 77.4,110.0 |
| **AUCINF(ng.h/mL)** | | | | | | |
| Ratio (%) | 112.9 | 101.2 | 138.2 | 111.6 | 81.7 | 73.2 |
| 90%CI | | | | | | |
| **Cmax (ng/mL)** | | | | | | |
| Ratio (%) | 82.3 | 81.6 | 88.3 | 100.8 | 93.2 | 92.5 |
| 90%CI | 73.3,92.3 | 72.6,91.8 | 78.6, 99.1 | 89.7,113.2 | 83.1, 104.5 | 82.4, 103.8 |
| **tmax** (h) | | | | | | |
| Difference | 0.48 | 0.51 | -0.05 | -0.03 | 0.53 | 0.56 |
| 90%CI | -0.22, 1.18 | -0.2, 1.23 | -0.76,0.66 | -0.74,0.68 | -0.17, 1.23 | -0.15, 1.27 |
| **t1/2Z (h)** | | | | | | |
| Difference | -1.46 | -1.70 | 2.48 | 0.24 | -3.94 | -4.18 |
| 90%CI | -5.332.40 | -5.72 2.32 | -1.266.23 | -3.614.08 | -7.51 -0.38 | -8.07 -0.29 |

**Table 19. Summary of Pharmacokinetic Parameters for Noroxymorphone by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 24 | 23 | 23 | 23 |
| Arithmetic Mean | 104.26 | 97.58 | 100.69 | 97.36 |
| (SD) | (37.930) | (35.393) | (37.876) | (35.559) |
| Geometric Mean | 94.39 | 88.51 | 91.01 | 87.67 |
| **AUCINF (ng.h/mL)** | | | | |
| N | 24 | 21 | 21 | 22 |
| Arithmetic Mean | 108.47 | 101.03 | 105.73 | 104.77 |
| (SD) | (38.451) | (37.666) | (36.655) | (33.155) |
| Geometric Mean | 98.86 | 91.47 | 97.1 | 97.17 |
| **Cmax (ng/mL)** | | | | |
| N | 24 | 23 | 23 | 23 |
| Arithmetic Mean | 5.36 | 4.97 | 5.16 | 4.90 |
| (SD) | (2.337) | (2.496) | (2.424) | (2.346) |
| Geometric Mean | 4.69 | 4.20 | 4.50 | 4.12 |
| **tmax (h)** | | | | |
| N | 24 | 23 | 23 | 23 |
| Median | 5.0 | 5.0 | 4.0 | 5.0 |
| (Min,Max) | (2.0, 12.0) | (3.0, 16.0) | (2.0, 12.0) | (1.5, 10.0) |
| **t1/2Z** | | | | |
| N | 24 | 21 | 21 | 23 |
| Arithmetic Mean | 10.82 | 10.04 | 10.37 | 10.32 |
| (SD) | (2.626) | (2.056) | (2.533) | (2.791) |
| **Noroxymorphone: Oxycodone** | | | | |
| **AUCt ratio (ng.h/mL)** | | | | |
| N | 24 | 23 | 23 | 23 |
| Arithmetic Mean | 0.23 | 0.21 | 0.22 | 0.20 |
| (SD) | (0.100) | (0.099) | (0.106) | (0.092) |
| **Noroxymorphone: Oxycodone** | | | | |
| **AUCINF ratio (ng.h/mL)** | | | | |
| N | 24 | 20 | 20 | 21 |
| Arithmetic mean | 0.24 | 0.21 | 0.23 | 0.21 |
| (SD) | (0.102) | (0.100) | (0.106) | (0.091) |

**Table 20. Noroxymorphone Summary of Ratios for AUCt, AUCINF, Cₘₐₓ and Differences for Tₘₐₓ and Half-Life - Full Analysis Population for Pharmacokinetics.**

| **Pharmacokinetic metric** | **4 x OXN 10/5 / 2 x Oxygesic 20 + 2 x Naloxone 10** | **2 x OXN 20/10 / 2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5 / 1 x OXN 40/20** | **2 x OXN 20/10 / 1 x OXN 40/20** |
|---|---|---|---|---|---|---|
| **AUCt (ng.h/mL)** | | | | | | |
| Ratio (%) | 102.9 | 98.4 | 101.2 | 104.5 | 101.6 | 97.2 |
| 90%CI | 99.0, 107.0 | 94.6, 102.4 | 97.4, 105.3 | 100.5,108.7 | 97.8, 105.6 | 93.5, 101.1 |
| **AUCINF(ng.h/mL)** | | | | | | |
| Ratio (%) | 102.7 | 99.3 | 100.7 | 103.4 | 102.0 | 98.6 |
| 90%CI | 98.7, 106.8 | 95.2, 103.5 | 96.6, 104.8 | 99.3, 107.7 | 98.0, 106.1 | 94.6, 102.8 |
| **Cmax (ng/mL)** | | | | | | |
| **Ratio (%)** | 108.9 | 97.8 | 104.6 | 111.4 | 104.1 | 93.4 |
| 90%CI | 95.3, 124.6 | 85.3,112.1 | 91.4,119.7 | 97.3, 127.5 | 91.1, 118.9 | 81.7, 106.9 |
| **tmax (h)** | | | | | | |
| Difference | 0.37 | 0.86 | 0.42 | -0.48 | -0.05 | 0.44 |
| 90%CI | -0.63, 1.37 | -0.16, 1.88 | -0.59, 1.43 | -1.49,0.52 | -1.04,0.95 | -0.57, 1.45 |
| **t1/2Z (h)** | | | | | | |
| Difference | 0.38 | -0.42 | -0.07 | 0.80 | 0.46 | -0.35 |
| 90%CI | -0.43, 1.20 | -1.29,0.45 | -0.93,0.78 | -0.05, 1.66 | -0.38, 1.30 | -1.22,0.53 |

**Table 21. Summary of Pharmacokinetic Parameters for 6-β Naloxol by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 24 | 23 | 23 | 23 |
| Arithmetic Mean | 13.16 | 12.39 | 13.55 | 13.77 |
| (SD) | (4.375) | (5.330) | (5.285) | (5.121) |
| Geometric Mean | 12.48 | 11.55 | 12.57 | 12.91 |
| **AUCINF (ng.h/mL)** | | | | |
| N | 13 | 15 | 16 | 19 |
| Arithmetic Mean | 13.38 | 13.85 | 14.24 | 15.07 |
| (SD) | (2.870) | (6.057) | (5.750) | (5.261) |
| Geometric Mean | 13.10 | 12.84 | 13.22 | 14.31 |
| **Cmax (ng/mL)** | | | | |
| N | 24 | 23 | 23 | 23 |
| Arithmetic Mean | 0.39 | 0.44 | 0.47 | 0.40 |
| (SD) | (0.175) | (0.352) | (0.238) | (0.206) |
| Geometric Mean | 0.37 | 0.38 | 0.43 | 0.37 |
| **tmax (h)** | | | | |
| N | 24 | 23 | 23 | 23 |
| Median | 1.0 | 0.5 | 8.0 | 2.5 |
| (Min,Max) | (0.5, 32.0) | (0.5, 32.0) | (0.5, 24.0) | (0.5, 36.0) |
| **t1/2Z** | | | | |
| N | 13 | 15 | 16 | 19 |
| Arithmetic Mean | 15.16 | 14.37 | 15.87 | 15.39 |
| (SD) | (1.906) | (3.459) | (5.607) | (5.340) |
| **6-β-Naloxol: Naloxone** | | | | |
| AUCt ratio (ng.h/mL) | | | | |
| N | 24 | 23 | 23 | 23 |
| Arithmetic Mean | 22.49 | 21.60 | 24.73 | 24.72 |
| (SD) | (14.103) | (18.348) | (24.359) | (25.824) |
| **6-β-Naloxol: Naloxone AUCINF ratio (ng.h/mL)** | | | | |
| N | 2 | 5 | 0 | 1 |
| Arithmetic mean | - | 9.79 | - | - |
| (SD) | - | (5.010) | - | - |

**Table 22. 6-β Naloxol Summary of Ratios for AUCt, AUCINF, Cₘₐₓ and Differences for Tₘₐₓ and Half-Life - Full Analysis Population for Pharmacokinetics.**

| **Pharmacokinetic metric** | **4 x OXN 10/5/2 x Oxygesic 20 + 2 x Naloxone 10** | **2 x OXN 20/10/2 x Oxygesic 20 + 2 x Naloxone 10** | **1 xOXN 40/20 / 2 x Oxygesic 20 + 2 x Naloxone 10** | **4 x OXN 10/5 / 2 x OXN 20/10** | **4 x OXN 10/5 / 1 x OXN 40/20** | **2 x OXN 20/10 / 1 x OXN 40/20** |
|---|---|---|---|---|---|---|
| **AUCt (ng.h/mL)** | | | | | | |
| Ratio (%) | 93.6 | 88.1 | 94.0 | 106.2 | 99.6 | 93.8 |
| 90%CI | 88.7,98.7 | 83.5, 93.1 | 89.1, 99.1 | 100.6,112.1 | 94.5, 105.0 | 88.9,99.0 |
| **AUCINF(ng.h/mL)** | | | | | | |
| **Ratio (%)** | 89.3 | 89.1 | 93.0 | 100.3 | 96.1 | 95.8 |
| 90%CI | 84.1,94.9 | 84.1,94.4 | 88.0,98.3 | 93.8, 107.2 | 90.2, 102.3 | 90.3, 101.6 |
| **Cmax (ng/mL)** | | | | | | |
| Ratio (%) | 97.8 | 103.0, | 113.8 | 95.0 | 85.9 | 90.5 |
| 90%CI | 86.4, 110.7 | 90.8, 116.9 | 100.5,128.9 | 83.8, 107.6 | 76.0,97.2 | 79.9, 102.5 |
| **tmax** (h) | | | | | | |
| Difference | -3.84 | -5.07 | -2.71 | 1.23 | -1.13 | -2.36 |
| 90%CI | -8.41,0.74 | -9.73, -0.41 | -7.32, 1.91 | -3.38,5.84 | -5.70,3.43 | -6.97,2.24 |
| **tl/2Z (h)** | | | | | | |
| Difference | -0.56 | -0.97 | 0.94 | 0.41 | -1.51 | -1.91 |
| 90%CI | -2.55, 1.43 | -2.90,0.96 | -0.90,2.79 | -1.79,2.60 | -3.59,0.58 | -3.89,0.06 |

### 5. Data Analysis

### a) Oxycodone Results

### - AUCt

The AUCt values obtained for oxycodone were very consistent between the treatments. Each of the treatments had a mean AUCt value of between 473 ng.h/mL (4 x OXN 10/5) and 502 ng.h/mL (2 x Oxygesic 20 mg & 2 x naloxone CR 10 mg).

In terms of AUCt, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

### - t1/2Z

The t1/2Z values obtained for oxycodone were consistent between the treatments. Each of the treatments had a mean t1/2Z value of between 4.69 h (4 x OXN 10/5), and 5.01 h (2 x Oxygesic 20 mg & 2 x naloxone CR 10 mg). There were no statistical differences between the t1/2Z values for the treatments for any of the comparisons that were made.

### - AUCINF

The AUCINF values obtained for oxycodone were very consistent between the treatments. Each of the treatments had a mean AUCINF value of between 475 ng.h/mL (4 x OXN 10/5) and 509 ng.h/mL (2 x Oxygesic 20 mg & 2 x naloxone CR 10 mg).

In terms of AUCINF, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

### - Cmax

The Cₘₐₓ values obtained for oxycodone were consistent between the fixed combination treatments, and ranged from 34.46 ng/mL (1 x OXN 40/20) to 35.73 ng/mL (2 x OXN 20/10). The mean Cₘₐₓ value for 2 x Oxygesic 20 mg & 2 x naloxone CR 10 mg was slightly higher at 40.45 ng/mL.

The Cₘₐₓ ratios comparing the fixed combination tablets with each other ranged from 97.5% to 103.1 %, and each had 90% confidence intervals within 80 - 125%. The higher mean Cₘₐₓ value for 2 x Oxygesic 20 mg & 2 x naloxone CR 10 mg meant that the Cₘₐₓ ratios comparing the fixed combination tablet with the reference product were lower, ranging from 85.8% to 88.4%. However, these Cₘₐₓ ratios were still associated with 90% confidence intervals that were within 80 - 125%.

### - tₘₐₓ

The median tₘₐₓ values for the fixed combination tablets ranged from 3 h (1 x OXN 40/20) to 4 h (2 x OXN 20/10). The difference between these two treatments, although apparently small, was statistically significant. The median tₘₐₓ for 2 x Oxygesic 20 mg & 2 x naloxone CR 10 mg was 2.5 h, and there was a statistically significant difference between this reference treatment and 2 x OXN 20/10.

### b) Naloxone-3-Glucuronide Results

### - AUCt

The AUCt values obtained for naloxone-3-glucuronide were very consistent between the treatments. Each treatment had a mean AUCt value of between 520 ng.h/mL (1 x OXN 40/20) and 540 ng.h/mL (4 x OXN 10/5).

In terms of AUCt, each of the fixed combination tablets provided an equivalent availability of naloxone-3-glucuronide to the reference treatment, and to each other. All of the relative bioavailability calculations had 90% confidence intervals that were within the 80 - 125% limits of acceptability for bioequivalence.

### - t½Z

The t½Z values obtained for naloxone-3-glucuronide were consistent between the treatments. Each of the treatments had a mean t½Z value of between 7.66 h (2 x Oxygesic 20 mg & 2 x naloxone CR 10 mg) and 8.48 h (4 x OXN 10/5). There were no statistical differences between the t1/2Z values for the treatments for any of the comparisons that were made.

### - AUCINF

The AUCINF values obtained for naloxone-3-glucuronide were very consistent between the treatments. Each of the treatments had a mean AUCINF value of between 521 ng.h/mL (2 x OXN 20/10) and 563 ng.h/mL (4 x OXN 10/5).

In terms of AUCINF, each of the fixed combination tablets provided an equivalent availability of naloxone-3-glucuronide to the reference treatment, and to each other. All of the bioavailability calculations had 90% confidence intervals that were within the 80 - 125% limits of acceptability for bioequivalence.

### - Cₘₐₓ

The Cₘₐₓ values obtained for naloxone-3-glucuronide were consistent between the treatments. Each of the treatments had a mean Cₘₐₓ value that range from 61.95 ng.mL (1 x OXN 40/20) to 63.62 ng.mL (2 x OXN 20/10).

Each of the fixed combination tablets provided an equivalent naloxone-3-glucuronide Cₘₐₓ to the reference treatment, and to each other. All of the Cₘₐₓ ratio calculations had 90% confidence intervals that were within the 80 - 125% limits of acceptability for bioequivalence.

### - tₘₐₓ

The median tₘₐₓ values for all the treatments ranged from 0.5 h (2 x OXN 20/10) to 1 h (4 x OXN 10/5, 1 x OXN 40/20 and 2 x Oxygesic 20 mg & 2 x naloxone CR 10 mg). There were no significant differences between the median tₘₐₓ values for any of the treatments.

### - Naloxone-3-glucuronide : naloxone AUCt ratios

The mean naloxone-3-glucuronide : naloxone AUCt ratios ranged from 852.25 (2 x Oxygesic 20 mg & 2 x naloxone CR 10 mg) to 933.46 (4 x OXN 10/5).

### - Naloxone-3-glucuronide : naloxone AUCINF ratios

The lack of AUCINF estimates for naloxone meant that mean naloxone-3-glucuronide : naloxone AUCINF ratios were only able to be calculated for 2 x OXN 20/10 tablets. These provided a mean naloxone-3-glucuronide : naloxone AUCINF ratio of 414.56, based on 5 subjects' data.

### d) Naloxone Results

Naloxone concentrations were low, as was anticipated; therefore these results did not support a full pharmacokinetic assessment.

### - AUCt

The AUCt values obtained for naloxone were consistent between the treatments. Each of the treatments had a mean AUCt value of between 0.84 ng.h/mL (2 x OXN 20/10) and 0.97 ng.h/mL (2 x Oxygesic 20 mg & 2 x naloxone CR 10 mg).

In terms of AUCt, each of the fixed combination tablets provided an equivalent availability of naloxone to the reference treatment, and to each other. All of the bioavailability calculations had 90% confidence intervals that were within the 80 - 125% limits of acceptability for bioequivalence.

### - t1/2Z

It was not possible to calculate t1/2Z values for naloxone for all of the subjects with confidence, because the plasma concentrations in the terminal part of the profile did not always approximate to a straight line when plotted on a semi-logarithmic scale. The mean values were based on numbers of subjects ranging from 4 to 9.

The mean tl/2Z values obtained for naloxone ranged from between 9.89 h (4 x OXN 10/5) to 13.83 h (1 x OXN 40/20). There were a wide range of t1/2Z values contributing to the means, however, there were no statistical differences between the t1/2Z values for the treatments for any of the comparisons that were made.

### - AUCINF

AUCINF values were calculated for those subjects with an estimable t1/2Z value. Some of the AUCINF values were not reportable because the extrapolated portion of the AUC accounted for more than 20% of the AUCINF value. A mean AUCINF value, of 1.64 ng.h/mL, was reportable for 2 x OXN 20/10 tablets only. None of the other treatments had sufficient data to report a mean AUCINF value. There were insufficient data to make comparisons between the treatments.

### - Cmax

Each of the treatments had a mean Cₘₐₓ value of between 0.07 ng/mL (4 x OXN 10/5) and 0.08 ng/mL (2 x OXN 20/10, 1 x OXN 40/20 and 2 x Oxygesic 20 mg & 2 x naloxone CR 10 mg).

Each of the fixed combination tablets provided an equivalent naloxone Cₘₐₓ to each other. All of the Cₘₐₓ ratios comparing the fixed combination tablets had 90% confidence intervals that were within the 80 - 125% limits of acceptability for bioequivalence.

When the fixed combination tablets were compared with the reference product, the 2 x OXN 20/10 tablets versus 2 x Oxygesic 20 mg & 2 x naloxone CR 10 mg had a 90% confidence interval that was above the 80 - 125% limit of acceptability for bioequivalence. The remaining fixed combination tablets provided an equivalent naloxone Cₘₐₓ to the reference product.

### - tₘₐₓ

The median tₘₐₓ values for the treatments ranged from 1 h (2 x Oxygesic 20 mg & 2 x naloxone CR 10 mg) to 5 h (2 x OXN 20/10). There were a wide range of tₘₐₓ values for each of the treatments. There were no significant differences between the median tₘₐₓ values for any of the treatments.

### e) Noroxycodone Results

### - AUCt

The AUCt values obtained for noroxycodone were very consistent between the treatments. Each of the treatments had a mean AUCt value of between 436 ng.h/mL (1 x OXN 40/20) and 451 ng.h/mL (2 x Oxygesic 20 mg & 2 x naloxone CR 10 mg).

In terms of AUCt, each of the AUCt, each of the fixed combination tablets provided an equivalent availability of noroxycodone to the reference treatment, and to each other. All of the relative bioavailability calculations had 90% confidence intervals that were within the 80 - 125% limits of acceptability for bioequivalence.

### - t1/2Z

The t1/2Z values obtained for noroxycodone were consistent between the treatments. Each of the treatments had a mean tl/2Z value of between 6.95 h (2 x Oxygesic 20 mg & 2 x naloxone CR 10 mg) and 7.25 h (1 x OXN 40/20). There were no statistical differences between the t1/2Z values for the treatments for any of the comparisons that were made.

### - AUCINF

The AUCINF values obtained for noroxycodone were very consistent between the treatments. Each of the treatments had a mean AUCINF value of between 441 ng.h/mL (1 x OXN 40/20) and 463 ng.h/mL (2 x Oxygesic 20 mg & 2 x naloxone CR 10 mg).

In terms of AUCINF, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

### - Cmax

The Cₘₐₓ values obtained for noroxycodone were consistent between treatments. Each of the treatments had a mean Cₘₐₓ value of between 24.26 ng/mL (1 x OXN 40/20) and 26.67 ng/mL (2 x Oxygesic 20 mg & 2 x naloxone CR 10 mg).

Each of the fixed combination tablets provided an equivalent noroxycodone Cₘₐₓ to the reference treatment, and to each other. All of the Cₘₐₓ ratio calculations had 90% confidence intervals that were within the 80 - 125% limits of acceptability for bioequivalence.

### - tₘₐₓ

The median tₘₐₓ values for the all the treatments ranged from 3.5 h to 5 h. There were no significant differences between the median tₘₐₓ values for any of the treatments.

### - Noroxycodone : oxycodone AUCt ratios

The mean noroxycodone : oxycodone AUCt ratios ranged from 0.91 (2 x OXN 20/10, 1 x OXN 40/20 and 2 x Oxygesic 20 mg & 2 x naloxone CR 10 mg) to 0.93 (4 x OXN 10/5).

### - Noroxycodone : oxycodone AUCINF ratios

The mean noroxycodone : oxycodone AUCt ratios ranged from 0.90 (1 x OXN 40/20) to 0.94 (4 x OXN 10/5).

### f) Oxymorphone Results

### - AUCt

The AUCt values obtained for oxymorphone were very consistent between treatments. Each of the treatments had a mean AUCt value of between 8 ng.h/mL (4 x OXN 10/5) and 9 ng.h/mL (1 x OXN 40/20).

In terms of AUCt, 4 x OXN 10/5 tablets and 1 x OXN 40/20 tablet provided an equivalent availability of oxymorphone to the reference treatment. 2 x OXN 20/10 tablets versus 2 x Oxygesic 20 mg & 2 x naloxone CR 10 mg had a 90% confidence interval that was outside the lower limit of acceptability for bioequivalence. When the fixed combination tablets were compared with each other, the 2 x OXN 20/10 tablets versus 1 x OXN 40/20 tablets had a 90% confidence interval outside the lower limit of acceptability for bioequivalence. The other comparisons between the fixed combination tablets had 90% confidence intervals that were within the 80 - 125% limits of acceptability for bioequivalence.

### - t1/2Z

It was not possible to calculate t1/2Z values for oxymorphone for all of the subjects with confidence, because the plasma concentrations in the terminal part of the profiles did not always approximate to a straight line when plotted on a semi-logarithmic scale. The mean values were based on numbers of subjects ranging from 9 for 2 x OXN 20/10 tablets to 14 for 4 x OXN 10/5 tablets. The mean t1/2Z values obtained for oxymorphone ranged between 10.66 h (2 x OXN 20/10) and 14.09 h (1 x OXN 40/20). There were no statistical differences between the half-life values for the fixed combination tablets and the reference product, however, the half-life value for 1 x OXN 40/20 was statistically longer than for the other two strengths of fixed combination tablets.

### - AUCINF

The mean AUCINF values were based on a small number of subjects for each of the treatments. AUCINF values could only be calculated for those subjects with an estimable t1/2Z value, and some AUCINF values were not reportable because the extrapolated portion of the AUC accounted for more than 20% of the AUCINF value. The numbers of subjects with reportable AUCINF values ranged from 4 for 4 x OXN 10/5 tablets and 1 x OXN 40/20 tablet, to 6 for 2 x Oxygesic 20 mg & 2 x naloxone CR 10 mg.

The mean AUCINF values ranged between 11 ng.h/mL (2 x Oxygesic 20 mg & 2 x naloxone CR 10 mg) and 18 ng.h/mL (1 x OXN 40/20). There were insufficient data to make comparisons between the treatments or calculate 90% confidence intervals.

### - Cₘₐₓ

Each of the treatments had a mean Cₘₐₓ value of between 0.57 ng/mL (4 x OXN 10/5) and 0.72 ng/mL (2 x Oxygesic 20 mg & 2 x naloxone CR 10 mg).

Each of the fixed combination tablets provided a lower oxymorphone Cₘₐₓ than the reference treatment. The 90% confidence intervals associated with the Cₘₐₓ ratios comparing the fixed combination tablets with the reference product were all below the lower limit of acceptability for bioequivalence.

Each of the fixed combination tablets provided an equivalent oxymorphone Cₘₐₓ to each other. All of the Cₘₐₓ ratios comparing the fixed combination tablets had 90% confidence intervals that were within the 80 - 125% limits of acceptability for bioequivalence.

### - tₘₐₓ

The median tₘₐₓ value for all of the treatments was 2 hours. There were no significant differences between the median tₘₐₓ values for any of the treatments.

### - Oxymorphone : oxycodone AUCt ratios

The mean oxymorphone : oxycodone AUCt ratios were 0.02 for all of the treatments.

### - Oxymorphone : oxycodone AUCINF ratios

The mean oxymorphone : oxycodone AUCINF ratios ranged from 0.02 (2 x OXN 20/10) to 0.03 (4 x OXN 10/5, 1 x OXN 40/20 and 2 x Oxygesic 20 mg & 2 x naloxone CR 10 mg).

### g) Noroxymorphone Results

### - AUCt

The AUCt values obtained for noroxymorphone were very consistent between treatments. Each of the treatments had a mean AUCt value of between 97 ng.h/mL (2 x Oxygesic 20 mg & 2 x naloxone CR 10 mg) and 104 ng.h/mL (4 x OXN 10/5).

In terms of AUCt, each of the fixed combination tablets provided an equivalent availability of noroxymorphone to the reference treatment, and to each other. Each of the bioavailability calculations had 90% confidence intervals that were within the 80 - 125% limits of acceptability for bioequivalence.

### - t1/2Z

The t1/2Z values obtained for noroxymorphone were consistent between the treatments. Each of the treatments had a mean t1/2Z value of between 10.04 h (2 x OXN 20/10) and 10.82 h (4 x OXN 10/5). There were no statistical differences between the t1/2Z values for the treatments for any of the comparisons that were made.

### - AUCINF

The AUCINF values obtained for noroxymorphone were very consistent between the treatments. Each of the treatments had a mean AUCINF value of between 101 ng.h/mL (2 x OXN 20/10) and 108 ng.h/mL (4 x OXN 10/5).

In terms of AUCINF, each of the fixed combination tablets provided an equivalent availability of noroxymorphone to the reference treatment, and to each other. All of the relative bioavailability calculations had 90% confidence intervals that were within the 80 - 125% limits of acceptability for bioequivalence.

### - Cmax

The Cₘₐₓ values obtained for noroxymorphone were consistent between the treatments. Each of the treatments had a mean Cₘₐₓ value that ranged from 4.90 ng/mL (2 x Oxygesic 20 mg & 2 x naloxone CR 10 mg) to 5.36 ng/mL (4 x OXN 10/5).

The Cₘₐₓ ratios comparing the fixed combination tablets with the reference product ranged from 97.8% to 108.9%, and each had 90% confidence intervals within 80 - 125%. When the fixed combination tablets were compared with each other, the 4 x OXN 10/5 tablets versus 2 x OXN 20/10 tablets had a 90% confidence interval outside the upper limit of acceptability for bioequivalence. The other comparisons between the fixed combination tablets had 90% confidence intervals that were within the 80 - 125% limits of acceptability for bioequivalence.

### - tₘₐₓ

The median tₘₐₓ values for the treatments ranged from 4 h to 5 h. There were no significant differences between the median tₘₐₓ values for any of the treatments.

### - Noroxymorphone : oxycodone AUCt ratios

The mean noroxymorphone : oxycodone AUCt ratios ranged from 0.20 (2 x Oxygesic 20 mg & 2 x naloxone CR 10 mg) to 0.23 (4 x OXN 10/5).

### - Noroxymorphone : oxycodone AUCINF ratios

The mean noroxymorphone : oxycodone AUCINF ratios ranged from 0.21 (2 x OXN 20/10 and 2 x Oxygesic 20 mg & 2 x naloxone CR 10 mg) to 0.24 (4 x OXN 10/5).

### h) 6β-Naloxol Results

### - AUCt

The AUCt values obtained for 6β-naloxol were very consistent between treatments. Each of the treatments had a mean AUCt value of between 12 ng.h/mL (2 x OXN 20/10) and 14 ng.h/mL (2 x Oxygesic 20 mg & 2 x naloxone CR 10 mg).

In terms of AUCt, each of the fixed combination tablets provided an equivalent availability of 6β-naloxol to the reference treatment, and to each other. Each of the bioavailability calculations had 90% confidence intervals that were within the 80 - 125% limits of acceptability for bioequivalence.

### - t1/2Z

The t1/2Z values obtained for 6β-naloxol were consistent between the treatments. Each of the mean treatments had a mean t1/2Z value of between 14.37 h (2 x OXN 20/10) and 15.87 h (1 x OXN 40/20). There were no statistical differences between the t1/2Z values for the treatments for any of the comparisons that were made.

### - AUCINF

The AUCINF values obtained for 6β-naloxol were very consistent between treatments. Each of the treatments had a mean AUCINF value of between 13 ng.mL (4 x OXN 10/5) and 15 ng.mL (2 x Oxygesic 20 mg & 2 x naloxone CR 10 mg).

In terms of AUCINF, each of the fixed combination tablets provided an equivalent availability of 6β-naloxol to the reference treatment and to each other. All of the relative bioavailability calculations had 90% confidence intervals that were within the 80 - 125% limits of acceptability for bioequivalence.

### - Cₘₐₓ

The mean Cₘₐₓ values obtained for 6β-naloxol for each of the treatments ranged from 0.39 ng/mL (4 x OXN 10/5) to 0.47 ng/mL (1 x OXN 40/20).

When the fixed combination tablets were compared with the reference product, 1 x OXN 40/20 tablet versus 2 x Oxygesic 20 mg & 2 x naloxone CR 10 mg had a 90% confidence interval that was above the upper limit of acceptability for bioequivalence. When the fixed combination tablets were compared with each other, the 4 x OXN 10/5 tablets versus 1 x OXN 40/20 tablet, and 2 x OXN 20/10 tablets versus 1 x OXN 40/20 tablet, both had 90% confidence intervals that were slightly below the lower limit of acceptability for bioequivalence. All remaining comparisons had 90% confidence intervals that were within the 80 - 125% limits of acceptability for bioequivalence.

### - tₘₐₓ

The median tₘₐₓ values for the treatments ranged from 0.5 h (2 x OXN 20/10) to 8 h (1 x OXN 40/20), and for each treatment, consisted of a wide range of individual tₘₐₓ values making up the median values. The median tₘₐₓ value for 2 x OXN 20/10 tablets was significantly lower than for 2 x Oxygesic 20 mg & 2 x naloxone CR 10 mg. There were no other significant differences between the median tₘₐₓ values for the remaining treatments.

### - 6β-naloxol : naloxone AUCt ratios

The mean 6β-naloxol : naloxone AUCt ratios ranged from 21.60 (2 x OXN 20/10) to 24.73 (1 x OXN 40/20).

### - 6β-naloxol : naloxone AUCINF ratios

The lack of AUCINF estimates for naloxone meant that mean 6β-naloxol :naloxone AUCINF ratios were only able to be calculated for 2 x OXN 20/10 tablets. These provided a mean 6β-naloxol : naloxone AUCINF ratio of 9.79, based on 5 subjects' data.

### 6. Clinical Pharmacology Discussion and Conclusions

Low oral bioavailability prevents the complete pharmacokinetic assessment of naloxone. This was confirmed as the low plasma concentrations meant that it was not possible to estimate AUCINF values for naloxone for most of the subjects. Naloxone-3-glucuronide was present in the plasma in much higher concentrations, and AUCINF estimates were obtained for naloxone-3-glucuronide for the majority of subjects. The conclusions for the naloxone component of the fixed combination tablets were based on naloxone-3-glucuronide parameters.

### a) Oxycodone

The mean plasma oxycodone concentration-time curves for 2 x Oxygesic 20 mg & 2 x naloxone CR 10 mg and the fixed combination tablets were almost superimposable.

A bioequivalence assessment was made for oxycodone. Each of the bioequivalence comparisons had 90% confidence intervals that were within the limits of acceptability for bioequivalence for Frelt, FrelINF and Cₘₐₓ ratio. The oxycodone results indicate that each of the fixed combination tablet strengths were bioequivalent, both to each other and also to Oxygesic given together with naloxone CR tablet. There were no statistical differences between any of the tₘₐₓ or t1/2Z values for any of the treatments, further confirming the similarity of the products.

The plasma oxycodone concentrations achieved after administration of the reference product were similar to dose-adjusted oxycodone concentrations seen after administration of OxyContin in a previous study. The mean Cₘₐₓ values for the fixed combination tablets were slightly lower, but when these were compared with the reference product, the Cₘₐₓ ratios had confidence intervals that were within the limits of acceptability for bioequivalence.

### b) Metabolite: parent AUCINF ratios

As expected, the levels of noroxycodone seen in the plasma after administration of the fixed combination tablets and Oxygesic plus naloxone, were similar to the levels of oxycodone that were achieved, resulting in noroxycodone : oxycodone AUCINF ratios of around 0.9. The levels of oxymorphone and noroxymorphone compared with oxycodone were much lower, with AUCINF ratios of around 0.02. These metabolite : parent AUCINF ratios were consistent across the fixed combination tablets and the reference treatment.

### c) Noroxycodone, oxymorphone and noroxymorphone

The noroxycodone data confirmed the oxycodone results. Each of the bioequivalence comparisons had 90% confidence intervals that were within the limits of acceptability for bioequivalence for Frelt, FrelINF and Cₘₐₓ ratio.

There were differences observed between the AUCt values for oxymorphone for 2 x OXN 20/10 versus 2 x Oxygesic 20 mg & 2 x naloxone CR 10 mg and 2 x OXN 20/10 versus 1 x OXN 40/20, however these differences were small, with only the lower limit of the 90% confidence interval being outside the limits of acceptability for bioequivalence. The fixed combination tablets were bioequivalent to each other in terms of Cₘₐₓ, but each provided a mean Cₘₐₓ value that was between 80% and 90% of the reference product Cₘₐₓ.

The noroxymorphone data also confirmed the oxycodone results. All but one of the bioequivalence comparisons had 90% confidence intervals that were within the limits of acceptability for bioequivalence for Frelt, FrelINF and Cₘₐₓ ratio.

### d) Naloxone

The mean plasma naloxone concentrations were low, less than 0.1 ng/mL, and appeared to be biphasic, with a second peak occurring at between 8 to 16 hours.

Even though all of the subjects did have quantifiable plasma naloxone concentrations, individual subjects' plasma naloxone concentrations were low and highly variable. The maximum observed plasma naloxone concentrations were 0.07 to 0.08 ng/mL.

The pharmacokinetic profiles of naloxone from earlier studies (OXU1001, OXU1004 and OXU9001) were examined. On average, the mean Cₘₐₓ values from these studies, dose-adjusted to a single dose of 1 mg, ranged between 4 and 15 pg/mL, confirming that the low plasma naloxone concentrations observed here were consistent with those levels measured in earlier studies.

A bioequivalence assessment was made for naloxone. The variability of the plasma naloxone concentrations did not allow for an estimate of AUCINF, or therefore FrelINF values. The bioavailability estimate was based on Frelt values. Each of the bioavailability comparisons had 90% confidence intervals that were within the limits of acceptability for bioequivalence. The mean Cₘₐₓ values for naloxone were comparable, and five out of the six bioavailability comparisons had 90% confidence intervals that met the criteria for bioequivalence.

The tₘₐₓ and t1/2Z values for the treatments were variable, however there were no significant differences between any of the treatments for these two parameters.

As expected, the levels of naloxone-3-glucuronide seen in the plasma after administration of the fixed combination tablets and Oxygesic plus naloxone, were much higher than the levels of naloxone that were achieved, resulting in naloxone-3-glucuronide : naloxone AUCt ratios of around 900. 6β-naloxol was also measured in higher quantities than naloxone, resulting in 6β-naloxol : naloxone AUCt ratios of around 22. These metabolite : parent AUCt ratios were consistent across the fixed combination tablets and the reference treatment.

### e) Naloxone-3-glucuronide

The mean plasma naloxone-3-glucuronide levels were higher than naloxone, and it was possible to make a bioavailability assessment based on FrelINF values.

A bioequivalence assessment was made for naloxone-3-glucuronide. Each of the bioequivalence comparisons had 90% confidence intervals that were within the limits of acceptability for bioequivalence for Frelt, FrelINF and Cₘₐₓ ratio. The naloxone-3glucuronide results indicate that each of the fixed combination tablet strengths were bioequivalent to each other, and to Oxygesic plus naloxone. There were no statistical differences between any of the tₘₐₓ or t1/2Z values for any of the treatments, further confirming the similarity of the products.

### f) 6β-naloxol

The 6β-naloxol data confirmed the naloxone and naloxone-3-glucuronide results. For most of the comparisons, there were no significant differences observed between the treatments and for the bioequivalence comparisons, most of the 90% confidence intervals were within the limits of acceptability for bioequivalence. There were small differences between the Cₘₐₓ values for the fixed combination products and the variability of the tₘₐₓ data led to a significant difference between the 2 x OXN 20/10 tablets and 2 x Oxygesic 20 mg & 2 x naloxone CR 10 mg.

### 7. Conclusion

These results confirm the interchangeability of the fixed combination tablets across the range of doses administered. This is supported by the bioavailability comparisons made between the treatments; each of the 90% confidence intervals for the ratio of population geometric means (test vs reference) for AUCINF and Cₘₐₓ of oxycodone and naloxone, fell within 80% - 125%. The fixed combination tablets were also shown to be bioequivalent to Oxygesic given together with naloxone CR tablet.

These data have also shown that the availability of oxycodone from the fixed combination tablets is similar to what we would expect from oxycodone given alone, indicating that the bioavailability of oxycodone is not influenced by the co-administration of naloxone.

Hence, the results may be summarized as follows:
- In terms of oxycodone and naloxone-3-glucuronide, each of the fixed combination tablet strengths are interchangeable.
- The fixed combination tablets were also shown to be bioequivalent to Oxygesic® + naloxone CR.
- There was no difference in the incidence of treatment-emergent adverse events between oxycodone and naloxone administered as a fixed OXN combination, and oxycodone and naloxone administered as an open combination.

Having thus described in detail preferred embodiments of the present invention, it is to be understood that the invention defined by the above paragraphs is not to be limited to particular details set forth in the above description, as many apparent variations thereof are possible without departing from the spirit or scope of the present invention.

All documents cited or referenced herein ("herein cited documents") including any manufacturer's instructions, descriptions, product specifications and product sheets for any products mentioned herein or in any document referenced herein, are hereby incorporated herein by reference. Citation or identification of any document in this application is not an admission that such document is available as prior art to the present invention. The detailed description, given by way of example, is not intended to limit the invention solely to the specific embodiments described.

## Claims

1. A dosage form comprising oxycodone and/or a pharmaceutically acceptable salt thereof and naloxone amd/or a pharmaceutically acceptable salt thereof, which provides a tₘₐₓ for oxycodone or a pharmaceutically acceptable salt at about 1 to about 17 hours, at about 2 to about 15 hours, at about 3 to about 8 hours or at about 4 to about 5 hours after administration to human patients.

2. The dosage form according to claim 1, which provides an improvement of bowel function during pain therapy, in particular an improvement of the mean bowel function score of at least about 5, at least about 8, at least about 10 or at least about 15 after administration to human patients, wherein the mean bowel function score is measured with a numerical analog scale ranging from 0 to 100.

3. The dosage form according to claim 1 or 2, which provides an analgesic effect for at least about 12 hours or at least about 24 hours after administration to human patients.

4. The dosage form according to any of the preceding claims, which provides an AUCt value for oxycodone of about 100 ng·h/mL to about 600 ng·h/mL, about 400 ng·h/mL to about 550 ng·h/mL, or about 450 to about 510 ng·h/mL.

5. The dosage form according to any of the preceding claims, which provides a Cₘₐₓ for oxycodone of about 5 ng/mL to about 50 ng/mL, about 30 ng/mL to about 40 ng/mL or about 35 ng/mL.

6. The dosage form according to any of the preceding claims,
wherein oxycodone and/or naloxone are released from the preparation in a sustained, invariant and/or independent manner.

7. The dosage form according to any of the preceding claims,
wherein oxycodone and/or naloxone are present in the form of a pharmaceutically acceptable salt.

8. The dosage form according to claim 7,
wherein oxycodone and/or naloxone are present in the form of a hydrochloride, sulfate, bisulfate, tartrate, nitrate, citrate, bitatrate, phosphate, malate, maleate, hydrobromide, hydroiodide, fumarate or succinate.

9. The dosage form according to any of the preceding claims,
wherein oxycodone or a pharmaceutically acceptable salt thereof is present in a unit dosage amount in excess of the unit dosage amount of naloxone.

10. The dosage form according to any of the preceding claims,
wherein naloxone or a pharmaceutically acceptable salt thereof is present in an amount of about 1 to about 50 mg, of about 5 to about 20 mg or of about 10 mg.

11. The dosage form according to any of the preceding claims,
wherein oxycodone or a pharmaceutically acceptable salt thereof is present in an amount of about 10 to about 150 mg, of about 20 to about 80 mg or of about 40 mg.

12. The dosage form according to any of the preceding claims,
wherein oxycodone or a pharmaceutically acceptable salt thereof and naloxone or a pharmaceutically acceptable salt thereof are present in weight ratio ranges of 25:1, 20:1, 15:1, 5:1, 4:1, 3:1, 2:1 or 1:1.

13. The dosage form according to any of the preceding claims,
wherein the preparation comprises a non-swellable and non-erosive diffusion matrix.

14. The dosage form according to claim 13,
wherein the diffusion matrix comprises at least one ethylcellulose component and at least one fatty alcohol.

15. The dosage form according to any of the preceding claims,
wherein the preparation contains fillers, lubricants, flowing agents and/or plasticizers.

16. The dosage form according to claim 15,
wherein the lubricant is selected from magnesium stearate, calcium stearate and/or calcium laureate and/or fatty acids, and is preferably stearic acid.

17. The dosage form according to claim 15 or 16,
wherein the flowing agent is selected from highly-disperse silica, preferably Aerosil®, Talcum, corn starch, magnesium oxide and magnesium stearate and/or calcium stearate.

18. The dosage form according to any of claims 14 to 17,
wherein the fatty alcohol is selected from lauryl, myrestyl, stearyl, cetostearyl, ceryl and/or cetyl alcohol, and is preferably stearyl alcohol.

19. The dosage form according to any of claims 14 to 18,
wherein the ethylcellulose component is a polymer mixture containing ethylcellulose.

20. The dosage form according to any of the preceding claims,
wherein the dosage form has been formulated for oral, nasal, rectal application and/or for application by inhalation.

21. The dosage form according to any of the preceding claims,
wherein the dosage form is a tablet, pill, capsule, granule and/or powder.

22. The dosage form according to any of the preceding claims,
wherein the dosage form or precursors thereof are produced by extrusion.

23. The dosage form according to any of the preceding claims, which is suitable for stable storage over a period of at least 2 years under standard conditions (60% relative humidity, 25°C) in accordance with admission guidelines.

24. Use of any of the dosage forms according to claims 1 to 23 for the preparation of a pharmaceutical preparation for pain treatment.

25. Use of any of the dosage forms according to claims 1 to 23 for the preparation of a pharmaceutical preparation for the treatment of pain and constipation during pain therapy.

26. Use of any of the dosage forms according to claims 1 to 23 for the preparation of a pharmaceutical preparation for the treatment of pain while preventing or reducing abuse.

27. Use according to any of claims 24 to 26, wherein the dosage form is suitable for once-a-day or twice-a-day administration at steady state or of a single dose to human patients.
